# NEW EUROPEAN PATENT SPECIFICATION

(11) **EP 1 727 808 B3**
(45) Date of publication of this specification: **09.01.2013**
(45) Mention of the grant of the patent: 12.11.2008
(21) Application number: 05717831.1
(22) Date of filing: 01.03.2005
(51) Int. Cl.: C07D 277/56, C07D 307/68, C07D 213/73, C07D 333/38, C07D 207/34, C07D 233/90, C07D 487/04, C07D 417/12, C07D 417/14, C07D 405/14, C07D 409/14, C07D 403/14, A61K 31/427, A61K 31/4025

(54) **BIARYL AMINO ACIDS AND THEIR USE IN DNA BINDING OLIGOMERS**
BIARYL AMINSÄURE UND IHRE VERWENDUNG IN DNS-BINDENDEN OLIGOMEREN
AMINOACIDES BIARYLES ET LEUR UTILISATION DANS LES OLIGOMERES QUI LIENT LE DNA

(30) Priority: 01.03.2004 GB 0404574
(43) Date of publication of application: 06.12.2006
(73) Proprietor: Spirogen Limited, Ryde, Isle of Wight PO33 2JF (GB)
(72) Inventor: HOWARD, Philip, Wilson, St Albans, Hertfordshire AL1 1LS (GB); WELLS, Geoffrey, Hull, Humberside HU11 4QG (GB)
(74) Representative: Watson, Robert James
(86) International application number: PCT/GB2005/000752
(87) International publication number: WO 2005/085177

(56) References cited:
- WO-A-99/46244
- US-A1- 2002 123 634
- A. PERJESSY ET. AL.: "Application of the Seth-Paul-Van Duyse Equation. III. Table 1" TETRAHEDRON, vol. 31, 1975, pages 2936-2939, XP002342320
- H. S. ANDERSEN ET. AL.: "Discovery and SAR of a Novel Selective and Orally Bioavailable Nonpeptide Classical Competitive Inhibitor of Protein Tyrosine Phosphate 1B." JOURNAL OF MEDICINAL CHEMISTRY, vol. 45, no. 20, 2002, pages 4443-4459, XP002342321
- R.L. DANNLEY ET. AL.: "Free Radical Aromatic Substitution. IV. The Reaction of Acyl Peroxides with Benzotrihalides." JOURNAL OF THE AMERICAN CHEMICAL SOCIETY, vol. 76, 20 September 1954 (1954-09-20), pages 4543-4545, XP009051912
- M. L. QUAN ET. AL.: "Biaryl Substituted Alkylboronate Esters as Thrombin Inhibitors" BIOORGANIC AND MEDICINAL CHEMISTRY LETTERS, vol. 7, no. 13, 1997, pages 1595-1600, XP002342322
- Y. QIAN ET. AL.: "Design and Synthesis of Non-Peptide Ras CAAX Mimetics as Potent Farnesyltransferase Inhibitors." JOURNAL OF MEDICINAL CHEMISTRY, vol. 39, 1996, pages 217-223, XP002342323
- C.R.WOODS ET. AL,: "Synthesis and DNA Binding Properties of Iminodiacetic Acid Linked Polyamides. Characterisation of Cooperative Extended 2:1 Side-by-Side Parallel Binding." JOURNAL OF THE AMERICAN CHEMICAL SOCIETY, vol. 124, 2002, pages 10676-10682, XP009051898 cited in the application
- C. A. BRIEHN ET. AL.: "Alternative Heterocycles for DNA Recognition: The Benzimidazole/Imidazole Pair." CHEMICAL EUROPEAN JOURNAL, vol. 9, 2003, pages 2110-2122, XP009051897 cited in the application
- S. J. GREGSON ET. AL.: "Synthesis of the First Examples of A-C8/C-C2 Amide-Linked Pyrrolo[2,1-c][1,4]benzodiazepine Dimers" BIOORGANIC AND MEDICINAL CHEMISTRY LETTERS, vol. 13, no. 14, 2003, pages 2277-2280, XP002342324

## Description

The present invention relates to amino acids, i.e. compounds bearing amino and carboxy groups, their synthesis and use in synthesizing molecules designed to interact with DNA.

### Background to the invention

The prototype minor groove binding agent distamycin A is a natural product with an amide linked tris(N-methylpyrrole) structure. The molecule binds non-covalently at A/T rich sequences and forms specific hydrogen bonds with the minor groove floor. The A/T recognising capacity of the molecule relates partly to favourable Van der Waals interactions with the groove walls in the relatively narrow A/T regions and also to specific steric clashes between the inner facing pyrrole H-3 and the larger G residues in the minor groove. The observation that distamycin and the related natural product netropsin may bind as highly cooperative 2:1 complexes in the minor groove was significant and prompted the development of a series of linked dimer molecules termed 'hairpin polyamides' (see for example, Woods, C.R., et al., J. Am. Chem. Soc., 124, 10676-10682 (2002)) In such molecules replacement of the pyrrole (Py) with the sterically less demanding imidazole (Im) allows passive G recognition. A further development was the inclusion of a hydroxypyrrole (Hp) unit which discriminated between T and A residues. Thus the full sequence recognising code is:

| Heterocycle | | Nucleotide | |
|---|---|---|---|
| Py | Py | A or T | A or T |
| Py | Hp | A | T |
| Hp | Py | T | A |
| Im | Py | G | C |
| Py | Im | C | G |

For molecules which bind in a 1:1 motif with DNA the recognition properties are more degenerate, thus:

| Heterocycle | Nucleotide |
|---|---|
| Py | A or T |
| Im | AorTorG orC |
| Hp | T? |

More recently new heterocycles have been studied such as 2-(pyrrol-2-yl)benzimidazoles, 2-(pyrrol-2-yl)imidazopyridines and 5-hydroxy-(pyrrol-2-yl)benzimidazoles which have similar recognition properties to the established building blocks in the context of hairpin polyamides (Biehen, C.A., et al., Chem. Eur. J., 9, 2110-2122 (2003)).

Some polyamide precursor molecules have been prepared previously. Methyl ester and 4-aminophenyl derivatives of 5-(3-aminophenyl)-furan-2-carboxylic acid have been synthesised by Perjessey et al. (Tetrahedron, 31, 2936-9, (1975)). 5-(3-aminophenyl)-oxalylamino-thiophene-2-carboxylic acid, its corresponding methyl ester and 5-(3-aminophenyl)-3-amino-thiophene-2-carboxylic acid are disclosed by Andersen et al. (J. Med. Chem., 45, 4443-4459, (2002)). 4'-amino-3-biphenylcarboxylic acid and 4'-amino-2-biphenylcarboxylic acid are disclosed by Dannley et al. (J. Am. Chem. Soc., 76, 4543-4545, (1954)). NH-Boc and NH-SO₂CH₃ protected derivatives of 3'-aminobiphenyl-3-carboxylic acid and 5-(3'-aminophenyl)-furan-2-carboxylic acid are disclosed by Quan et al. (Bioorg. & Med. Chem. Lett., 7, 1595-1600, (1997)). The t-Butyl ester derivative of 4'-aminobiphenyl-3-carboxylic acid is disclosed by Qian et al. (J. Med. Chem., 39, 217-223, (1996)). WO 99/046244 discloses 5-(4-aminophenyl)-3-oxalylamino-thiophene-2-carboxylic acid.

### Disclosure of the invention

The present inventors have developed a series of compounds bearing amino and carboxy groups, which can be used in synthesising molecules designed to interact with DNA.

In a first aspect, the invention provides a compound of formula I:

Z'-CO-A-B-NH-Z (I)

wherein:
(A) Z is an amino protecting group;
   Z' is OH, a protected or activated hydroxyl group or Cl;
   A is an optionally substituted C₅₋₆ arylene group;
   B is an optionally substituted C₅₋₆ arylene group;
   And wherein one of A and B is phenylene and the other of A and B is a C5-heteroarylene group, wherein when B is phenylene with an -NH- β to the bond between A and B, then -A-CO- is not: 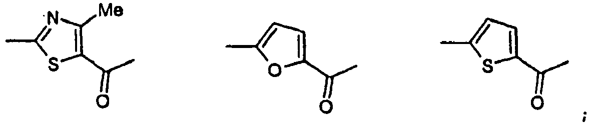 or
(B) Z is H;
   Z' is OH, a protected or activated hydroxyl group or Cl;
   A is an optionally substituted C₅₋₆ arylene group;
   B is an optionally substituted C₅₋₆ arylene group;
   and wherein one of A and B is phenylene and the other of A and B is a C5-heteroarylene group, and if B is phenylene, then A is not thiazolylene, furnaylene or thiophenylene, and if B is pyridylene, then A is not phenylene.

In a second aspect, the invention provides a method of synthesising a compound of formula I.

In a third aspect of the invention, there are provided compounds are of formula IV:

Z"-(T)ₙ-CO-(CH₂)_{q}-NR¹R² (IV)

comprising a polyamido moiety wherein:
Z" is OH or a protected hydroxy group;
each T is independently selected from units of formulae II, III or V:

   -CO- A- B- NH- (II)

   -CO-E-NH- (III)

   -CO-(CH₂)_{q'}-NH- (V)
and at least one T is a unit of formula II;
wherein q' is from 1 to 3;
n is from 1 to 10;
A and B are as defined in the first aspect of the invention.
E is either optionally substituted C₅₋₂₀ heteroarylene (G) or C₈₋₁₀ heteroarylene-C₅₋₂₀ arylene (K)
q is from 1 to 3; and
R¹ and R² are independently selected from C₁₋₄ alkyl.

In a fourth aspect of the invention, there are provided compounds comprising a polyamide moiety, itself comprising at least one unit of formula II:

-CO- A- B- NH- (II)

, wherein A and B are as defined in the first aspect of the invention, and further include a pyrrolobenzodiazepine moiety of formula VI: 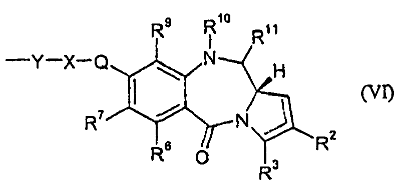 wherein:
the dotted lines indicate the optional presence of a double bond between C1 and C2 or C2 and C3;
R² and R³ are independently selected from -H, -OH, =O, =CH₂, -CN,-R, OR, halo, =CH-R, O-SO₂-R, CO₂R and COR;
R⁶, R⁷ and R⁹ are independently selected from H, R, OH, OR, SH, SR, NH₂, NHR, NRR', nitro, Me₃Sn and halo;
where R and R' are independently selected from optionally substituted C₁₋₇ alkyl, C₃₋₂₀ heterocyclyl and C₅₋₂₀ aryl groups; or
R⁶ and R⁷ together form a group -O-(CH₂)ₚ-O-, where p is 1 or 2;
A¹⁰ is a nitrogen protecting group and R¹¹ is either O-R¹⁵, wherein R¹⁵ Is a hydroxyl protecting group, or R¹¹ is OH, or R¹⁰ and R¹¹ together form a double bond between N10 and C11;
Q is selected from O, S, NH or a single bond;
X is a divalent group such that HY = R, or a single bond;
Y is either NH or C(=O).

Further aspects of the present invention relate to compounds of the third and fourth aspects of the invention and pharmaceutical salts thereof, their use in methods of therapy (particularly in treating gene-based diseases), pharmaceutical compositions comprising these, and their use in the manufacture of a medicament for the treatment of a gene-based disease.

### Definitions

### C₅₋₆ arylene groups

The term C₅₋₆ arylene, as used herein, pertains to a divalent moiety obtained by removing two hydrogen atoms from aromatic ring atoms of an aromatic compound and 5 or 6 ring atoms.

The ring atoms may be all carbon atoms, as in "carboaryl groups". Examples of carboaryl groups include, but are not limited to, those derived from benzene (i.e. phenylene) (C₆).

Alternatively, the ring atoms may include one or more heteroatoms, as in "C₅₋₆ heteroarylene groups". Examples of C₅₋₆ heteroarylene groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅);
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine)
(C₆) (e.g., cytosine, thymine, uracil), pyrazine (1,4-diazine) (C₆);
N₃: triazole (C₅), triazine (C₆); and,
N₄: tetrazole (C₅).
*C₅₋₂₀ heteroarylene groups (G)*

G is an optionally substituted C₅₋₂₀ heteroarylene group, preferably a C₅₋₁₆ heteroarylene group, more preferably a C₅₋₁₀ heteroarylene group and even more preferably a C₅₋₆ heteroarylene group.

Furthermore in a preferred embodiment, the G group is a five membered heteroarylene group.

The heteroarylene group may contain one or more heteroatoms and preferably contains one heteroatom. The heteroatoms in the heteroarylene group are independently chosen from N, O and S and are preferably N.

The heteroarylene G group is optionally substituted with one or more R groups. In a preferred embodiment the G group is substituted at one or more of the heteroatom positions with at least one R group, most preferably the R group is a methyl or ethyl group.

Where the G group is a six membered heteroarylene group, the -NH- and -CO- groups are preferably attached at the 2,6, 2,5, 3,6 or 3,5 positions.

Where the G group is a five membered heteroarylene group, the -NH- and -CO- groups are preferably attached at the 2,5, 2,4 or 3,5 positions.

Where the G group comprises two fused rings, the -NH- and -CO- groups are preferably attached to different rings.

Known amino-C₅ heteroarylene-carbonyl units include the following:

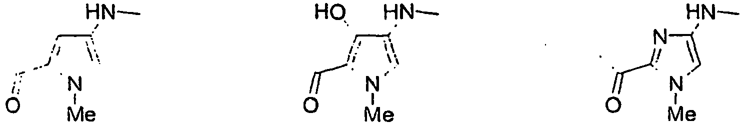

### C₈₋₁₀ heteroarylene-C₅₋₂₀ arylene groups (K)

The C₈₋₁₀ heteroarylene groups are a subset of the C₅₋₂₀ heteroarylene groups defined above, and comprise two fused rings.

The term arylene, as used herein, pertains to a divalent moiety obtained by removing two hydrogen atoms from aromatic ring atoms of an aromatic compound having from 5 to 20 ring atoms. Arylene compounds as described herein correspond to aryl groups as defined below with one fewer hydrogen atoms on the ring atoms.
Preferably, the C₅₋₂₀ arylene group is a C₅₋₇ arylene group and more preferably a C₅₋₆ heteroarylene group.

Units of formula III which are a carbonyl-C₈₋₁₀ heteroarylene-C₅₋₆ heteroarylene-amino unit have been described in Briehen, C.A., et al., Chem. Eur. J., 9,2110-2122 (2003) and Renneberg, D., et al., J. Am. Chem. Soc., 125, 5707-5716 (2003) and include:

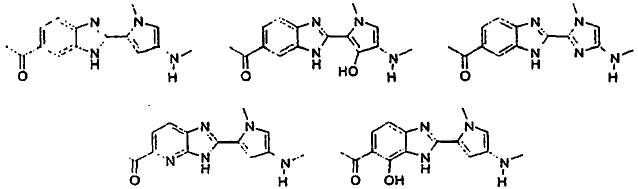

### Amino protecting groups (Z)

Amino protecting groups are well known in the art, and are listed on pages 494 to 572 of Greene, T.W. and Wuts, G.M., Protective Groups in Organic Synthesis, 3rd Edition, John Wiley & Sons, Inc., 1999, which is incorporated herein by reference. Preferred nitrogen protecting groups are carbamate protecting groups that have the general formula:

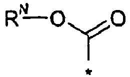

Particularly preferred protecting groups include Alloc, Troc, Teoc, Boc, and Fmoc, with Boc being particularly preferred.

### Protected hydroxyl groups (Z')

Protected hydroxyl groups are of the formula -O-Prot, where Prot is an oxygen protecting group as discussed below.

### Activated hydroxyl groups (Z')

Activated hydroxyl groups are of the formula -O-Act, where Act is an activating moiety for peptide bond formation, introduced by a peptide coupling reagent. Such reagents include BOP, BOP-Cl, DCC, DIC, EDPP, HATU, HOBt, PyBroP and TBTU.

### Nitrogen protecting groups (R¹⁰)

Nitrogen protecting groups are well known in the art. Preferred nitrogen protecting groups are carbamate protecting groups that have the general formula:

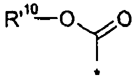

A large number of possible carbamate nitrogen protecting groups are listed on pages 503 to 549 of Greene, T.W. and Wuts, G.M., Protective Groups in Organic Synthesis, 3rd Edition, John Wiley & Sons, Inc., 1999, which is incorporated herein by reference.

Particularly preferred protecting groups include Alloc, Troc, Teoc, BOC, Doc, Hoc, TcBOC, Fmoc, 1-Adoc and 2-Adoc.

Also suitable for use in the present invention are nitrogen protecting groups which can be removed *in vivo* (e.g. enzymatically, using light) as described in WO 00/12507, which is incorporated herein by reference. Examples of these protecting groups include: 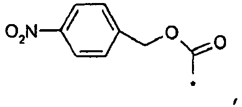 which is nitroreductase labile (e.g. using ADEPT/GDEPT); 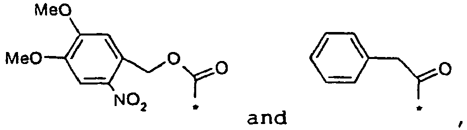 which are photolabile; and 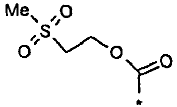 which is glutathione labile (e.g. using NPEPT).

### Oxygen protecting groups

Oxygen protecting groups are well known in the art. A large number of suitable groups are described on pages 23 to 200 of Greene, T.W. and Wuts, G.M., Protective Groups in Organic Synthesis, 3rd Edition, John Wiley & Sons, Inc., 1999, which is incorporated herein by reference.

Classes of particular interest include silyl ethers, methyl ethers, alkyl ethers, benzyl ethers, esters, benzoates, carbonates, and sulfonates.

### Substituents

The phrase "optionally substituted" as used herein, pertains to a parent group which may be unsubstituted or which may be substituted.

Unless otherwise specified, the term "substituted" as used herein, pertains to a parent group which bears one or more substitutents. The term "substituent" is used herein in the conventional sense and refers to a chemical moiety which is covalently attached to, or if appropriate; fused to, a parent group. A wide variety of substituents are well known, and methods for their formation and introduction into a variety of parent groups are also well known.

Examples of substituents are described in more detail below.

C₁₋₇ alkyl: The term "C₁₋₇ alkyl" as used herein, pertains to a monovalent moiety obtained by removing a hydrogen atom from a carbon atom of a hydrocarbon compound having from 1 to 7 carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

Examples of saturated alkyl groups include, but are not limited to, methyl (C₁), ethyl (C₂), propyl (C₃), butyl (C₄), pentyl (C₅), hexyl (C₆) and heptyl (C₇).

Examples of saturated linear alkyl groups include, but are not limited to, methyl (C₁), ethyl (C₂), n-propyl (C₃), n-butyl (C₄), n-pentyl (amyl) (C₅), n-hexyl (C₆) and n-heptyl (C₇).

Examples of saturated branched alkyl groups include iso-propyl (C₃), iso-butyl (C₄), sec-butyl (C₄), tert-butyl (C₄), iso-pentyl (C₅), and neo-pentyl (C₅).

C₂₋₇ Alkenyl: The term "C₂₋₇ alkenyl" as used herein, pertains to an alkyl group having one or more carbon-carbon double bonds.

Examples of unsaturated alkenyl groups include, but are not limited to, ethenyl (vinyl, -CH=CH₂). 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₇ alkynyl: The term "C₂₋₇ alkynyl" as used herein, pertains to an alkyl group having one or more carbon-carbon triple bonds.

Examples of unsaturated alkynyl groups include, but are not limited to, ethynyl (ethinyl, -C≡CH) and 2-propynyl (propargyl, - CH₂-C≡CH).

C₃₋₇ cycloalkyl: The term "C₃₋₇ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) and methylcyclohexane (C₇);
unsaturated monocyclic hydrocarbon compounds:
   cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbomane (C₇).

C₃₋₂₀ heterocyclyl: The term "C₃₋₂₀ heterocyclyl" as used herein, pertains to a monovalent moiety obtained by removing a hydrogen atom from a ring atom of a heterocyclic compound, which moiety has from 3 to 20 ring atoms, of which from 1 to 10 are ring heteroatoms. Preferably, each ring has from 3 to 7 ring atoms, of which from 1 to 4 are ring heteroatoms.

In this context, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidirie (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Examples of substituted monocyclic heterocyclyl groups include those derived from saccharides, in cyclic form, for example, furanoses (C₅), such as arabinofuranose, lyxofuranose, ribofuranose, and xylofuranse, and pyranoses (C₆), such as allopyranose, altropyranose, glucopyranose, mannopyranose, gulopyranose, idopyranose, galactopyranose, and talopyranose.

C₅₋₂₀ aryl: The term "C₅₋₂₀ aryl", as used herein, pertains to a monovalent moiety obtained by removing a hydrogen atom from an aromatic ring atom of an aromatic compound, which moiety has from 3 to 20 ring atoms. Preferably, each ring has from 5 to 7 ring atoms.

In this context, the prefixes (e.g. C₃₋₂₀, C₅₋₇, C₅₋₆, etc.) denote the number of ring atoms, or range of number of ring atoms, whether carbon atoms or heteroatoms. For example, the term "C₅₋₆ aryl" as used herein, pertains to an aryl group having 5 or 6 ring atoms.

The ring atoms may be all carbon atoms, as in "carboaryl groups". Examples of carboaryl groups include, but are not limited to, those derived from benzene (i.e. phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic ring, include, but are not limited to, groups derived from indane (e.g. 2,3-dihydro-1H-indene) (C₉), indene (C₉), isoindene (C₉), tetraline (1,2,3.4-tetrahydronaphthalene (C₁₀), acenaphthene (C₁₂), fluorene (C₁₃), phenalene (C₁₃), acephenanthrene (C₁₅), and aceanthrene (C₁₆).

Alternatively, the ring atoms may include one or more heteroatoms, as in "heteroaryl groups". Examples of monocyclic heteroaryl groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅);
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine)
(C₆) (e.g., cytosine, thymine, uracil), pyrazine (1,4-diazine) (C₆);
N₃: triazole (C₅), triazine (C₆); and,
N₄: tetrazole (C₅).

Examples of heteroaryl which comprise fused rings, include, but are not limited to:
C₉ (with 2 fused rings) derived from benzofuran (O₁), isobenzofuran (O₁), indole (N₁), isoindole (N₁), indolizine (N₁), indoline (N₁), isoindoline (N₁), purine (N₄) (e.g., adenine, guanine), benzimidazole (N₂), indazole (N₂), benzoxazole (N₁O₁), benzisoxazole (N₁O₁), benzodioxole (O₂), benzofurazan (N₂O₁), benzotriazole (N₃), benzothiofuran (S₁), benzothiazole (N₁S₁), benzothiadiazole (N₂S);
C₁₀ (with 2 fused rings) derived from chromene (O₁), isochromene (O₁), chroman (O₁), isochroman (O₁), benzodioxan (O₂), quinoline (N₁), isoquinoline (N₁), quinolizine (N₁), benzoxazine (N₁O₁), benzodiazine (N₂), pyridopyridine (N₂), quinoxaline (N₂), quinazoline (N₂), cinnoline (N₂), phthalazine (N₂), naphthyridine (N₂), pteridine (N₄);
C₁₁ (with 2 fused rings) derived from benzodiazepine (N₂);
C₁₃ (with 3 fused rings) derived from carbazole (N₁), dibenzofuran (O₁), dibenzothiophene (S₁), carboline (N₂), perimidine (N₂), pyridoindole (N₂); and,
C₁₄ (with 3 fused rings) derived from acridine (N₁), xanthene (O₁), thioxanthene (S₁), oxanthrene (O₂), phenoxathiin (O₁S₁), phenazine (N₂), phenoxazine (N₁O₁), phenothiazine (N₁S₁), thianthrene (S₂), phenanthridine (N₁), phenanthroline (N₂), phenazine (N₂).

The above groups, whether alone or part of another substituent, may themselves optionally be substituted with one or more groups selected from themselves and the additional substituents listed below.

Halo: -F, -Cl, -Br, and -I.

Hydroxy: -OH.

Ether: -OR, wherein R is an ether substituent, for example, a C₁₋₇ alkyl group (also referred to as a C₁₋₇ alkoxy group, discussed below), a C₃₋₂₀ heterocyclyl group (also referred to as a C₃₋₂₀ heterocyclyloxy group), or a C₅-₂₀ aryl group (also referred to as a C₅₋₂₀ aryloxy group), preferably a C₁₋₇alkyl group.

Alkoxy: -OR, wherein R is an alkyl group, for example, a C₁₋₇ alkyl group. Examples of C₁₋₇ alkoxy groups include, but are not limited to, -OMe (methoxy), -OEt (ethoxy), -O(nPr) (n-propoxy), -O(iPr) (isopropoxy), -O(nBu) (n-butoxy), -O(sBu) (sec-butoxy), -O(iBu) (isobutoxy), and -O(tBu) (tert-butoxy).

Acetal: -CH(OR¹)(OR²), wherein R¹ and R² are independently acetal substituents, for example, a C₁₋₇ alkyl group, a C₃₋₂₀ heterocyclyl group, or a C₅₋₂₀aryl group, preferably a C₁₋₇ alkyl group, or, in the case of a "cyclic" acetal group, R¹ and R², taken together with the two oxygen atoms to which they are attached, and the carbon atoms to which they are attached, form a heterocyclic ring having from 4 to 8 ring atoms. Examples of acetal groups include, but are not limited to, -CH(OMe)₂, -CH(OEt)₂, and -CH(OMe)(OEt).

Hemiacetal: -CH(OH) (OR¹), wherein R¹ is a hemiacetal substituent, for example, a C₁₋₇ alkyl group, a C₃₋₂₀ heterocyclyl group, or a C₅₋₂₀ aryl group, preferably a C₁₋₇ alkyl group. Examples of hemiacetal groups include, but are not limited to, -CH(OH)(OMe) and - CH(OH)(OEt).

Ketal: -CR(OR¹)(OR²), where R¹ and R² are as defined for acetals, and R is a ketal substituent other than hydrogen, for example, a C₁₋₇ alkyl group, a C₃₋₂₀ heterocyclyl group, or a C₅₋₂₀ aryl group, preferably a C₁₋₇ alkyl group. Examples ketal groups include, but are not limited to, -C(Me)(OMe)₂, -C(Me)(OEt)₂, -C(Me)(OMe)(OEt), - C(Et)(OMe)₂, -C(Et)(OEt)₂, and -C (Et) (OMe)(OEt) .

Hemiketal: -CR(OH)(OR¹), where R¹ is as defined for hemiacetals, and R is a hemiketal substituent other than hydrogen, for example, a C₁₋₇ alkyl group, a C₃₋₂₀ heterocyclyl group, or a C₅₋₂₀ aryl group, preferably a C₁₋₇ alkyl group. Examples of hemiacetal groups include, but are not limited to, -C(Me)(OH)(OMe), -C(Et)(OH)(OMe), -C (Me) (OH)(OEt), and -C (Et) (OH)(OEt).

Oxo (keto, -one): =O.

Thione (thioketone): =S.

Imino (imine): =NR, wherein R is an imino substituent, for example, hydrogen, C₁₋₇ alkyl group, a C₃₋₂₀ heterocyclyl group, or a C₅₋₂₀ aryl group, preferably hydrogen or a C₁₋₇ alkyl group. Examples of ester groups include, but are not limited to, =NH, =NMe, =NEt, and =NPh.

Formyl (carbaldehyde, carboxaldehyde): -C(=O)H.

Acyl (keto): -C(=O)R, wherein R is an acyl substituent, for example, a C₁₋₇ alkyl group (also referred to as C₁₋₇ alkylacyl or C₁₋₇ alkanoyl), a C₃₋₂₀ heterocyclyl group (also referred to as C₃₋₂₀ heterocyclylacyl), or a C₅₋₂₀ aryl group (also referred to as C₅₋₂₀ arylacyl), preferably a C₁₋₇ alkyl group. Examples of acyl groups include, but are not limited to, -C(=O)CH₃ (acetyl), -C(=O)CH₂CH₃ (propionyl), -C(=O)C(CH₃)₃ (t-butyryl), and -C(=O)Ph (benzoyl, phenone).

Carboxy (carboxylic acid): -C(=O)OH.

Thiocarboxy (thiocarboxylic acid): -C(=S)SH.

Thiolocarboxy (thiolocarboxylic acid): -C(=O)SH.

Thionocarboxy (thionocarboxylic acid): -C(=S)OH.

Imidic acid: -C(=NH)OH.

Hydroxamic acid: -C(=NOH)OH.

Ester (carboxylate, carboxylic acid ester, oxycarbonyl): -C(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -C(=O)OCH₃, -C(=O)OCH₂CH₃, -C(=O)OC(CH₃)₃, and -C(=O)OPh.

Acyloxy (reverse ester): -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Examples of acyloxy groups include, but are not limited to, -OC(=O)CH₃ (acetoxy), -OC(=O)CH₂CH₃, -OC(=O)C(CH₃)₃, -OC(=O)Ph, and -OC(=O)CH₂Ph.

Oxycarboyloxy: -OC(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -OC(=O)OCH₃, -OC(=O)OCH₂CH₃, -OC(=O)OC(CH₃)₃, and -OC(=O)OPh.

Amino: -NR¹R², wherein R¹ and R² are independently amino substituents, for example, hydrogen, a C₁₋₇ alkyl group (also referred to as C₁₋₇ alkylamino or di-C₁₋₇ alkylamino), a C₃₋₂₀ heterocyclyl group, or a C₅₋₂₀ acryl group, preferably H or a C₁₋₇ alkyl group, or, in the case of a "cyclic" amino group, R¹ and R², taken together with the nitrogen atom to which they are attached, form a heterocyclic ring having from 4 to 8 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to, -NH₂, -NHCH₃, -NHC(CH₃)₂, -N(CH₃)₂, -N(CH₂CH₃)₂, and -NHPh. Examples of cyclic amino groups include, but are not limited to, aziridino, azetidino, pyrrolidino, piperidino, piperazino, morpholino, and thiomorpholino.

Amido (carbamoyl, carbamyl, aminocarbonyl, carboxamide): -C(=O)NR¹R², wherein R¹ and R² are independently amino substituents, as defined for amino groups. Examples of amido groups include, but are not limited to, -C(=O) NH₂, -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Thioamido (thiocarbamyl): -C(=S)NR¹R², wherein R¹ and R² are independently amino substituents, as defined for amino groups. Examples of amido groups include, but are not limited to, -C(=S)NH₂, -C(=S)NHCH₃, -C(=S)N(CH₃)₂, and -C(=S)NHCH₂CH₃.

Acylamido (acylamino) : -NR¹C(=O)R², wherein R¹ is an amide substituent, for example, hydrogen, a C₁₋₇ alkyl group, a C₃₋₂₀ heterocyclyl group, or a C₅₋₂₀ aryl group, preferably hydrogen or a C₁₋₇ alkyl group, and R² is an acyl substituent, for example, a C₁₋₇ alkyl group, a C₃₋₂₀ heterocyclyl group, or a C₅₋₂₀aryl group, preferably hydrogen or a C₁₋₇ alkyl group. Examples of acylamide groups include, but are not limited to, -NHC(=O)CH₃ , -NHC(=O)CH₂CH₃, and -NHC(=O)Ph. R¹ and R² may together form a cyclic structure, as in, for example, succinimidyl, maleimidyl, and phthalimidyl:

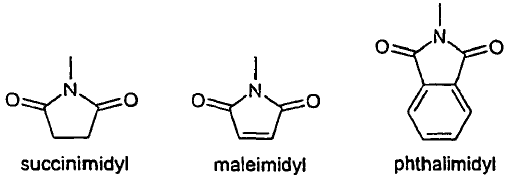

Aminocarbonyloxy: -OC(=O)NR¹R², wherein R¹ and R² are independently amino substituents, as defined for amino groups. Examples of aminocarbonyloxy groups include, but are not limited to, -OC(=O)NH₂, -OC(=O)NHMe, -OC (=O)NMe₂, and -OC(=O)NEt₂.

Ureido: -N(R¹)CONR²R³ wherein R² and R³ are independently amino substituents, as defined for amino groups, and R¹ is a ureido substituent, for example, hydrogen, a C₁₋₇ alkyl group, a C₃₋₂₀ heterocyclyl group, or a C₅₋₂₀ aryl group, preferably hydrogen or a C₁₋₇ alkyl group. Examples of ureido groups include, but are not limited to, -NHCONH₂, -NHCONHMe, -NHCONHEt, -NHCONMe₂, -NHCONEt₂, - NMeCONH₂, -NMeCONHMe, -NMeCONHEt, -NMeCONMe₂, and -NMeCONEt₂,

Guanidino: -NH-C(=NH)NH₂.

Tetrazolyl: a five membered aromatic ring having four nitrogen atoms and one carbon atom,

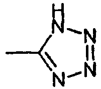

Imino: =NR, wherein R is an imino substituent, for example, for example, hydrogen, a C₁₋₇ alkyl group, a C₃₋₂₀ heterocyclyl group, or a C₅₋₂₀ aryl group, preferably H or a C₁₋₇alkyl group. Examples of imino groups include, but are not limited to, =NH, =NMe, and =NEt.

Amidine (amidino): -C(=NR)NR₂, wherein each R is an amidine substituent, for example, hydrogen, a C₁₋₇ alkyl group, a C₃₋₂₀ heterocyclyl group, or a C₁₋₂₀ aryl group, preferably H or a C₁₋₇ alkyl group. Examples of amidine groups include, but are not limited to, -C(=NH)NH₂, -C(=NH)NMe₂, and -C(=NMe)NMe₂.

Nitro: -NO₂.

Nitroso: -NO.

Azido: -N₃.

Cyano (nitrile, carbonitrile): -CN.

Isocyano: -NC.

Cyanato: -OCN.

Isocyanato: -NCO.

Thiocyano (thiocyanato): -SCN.

Isothiocyano (isothiocyanato): -NCS.

Sulfhydryl (thiol, mercapto): -SH.

Thioether (sulfide): -SR, wherein R is a thioether substituent, for example, a C₁₋₇ alkyl group (also referred to as a C₁₋₇alkylthio group), a C₃₋₂₀ heterocyclyl group, or a C₅₋₂₀ aryl group, preferably a C₁₋₇ alkyl group. Examples of C₁₋₇ alkylthio groups include, but are not limited to, -SCH₃ and -SCH₂CH₃.

Disulfide: -SS-R, wherein R is a disulfide substituent, for example, a C₁₋₇ alkyl group, a C₃₋₂₀ heterocyclyl group, or a C₅₋₂₀ aryl group, preferably a C₁₋₇ alkyl group (also referred to herein as C₁₋₇ alkyl disulfide). Examples of C₁₋₇ alkyl disulfide groups include, but are not limited to, -SSCH₃ and -SSCH₂CH₃.

Sulfine (sulfinyl, sulfoxide): -S(=O)R, wherein R is a sulfine substituent, for example, a C₁₋₇ alkyl group, a C₃₋₂₀ heterocyclyl group, or a C₅₋₂₀ aryl group, preferably a C₁₋₇ alkyl group. Examples of sulfine groups include, but are not limited to, -S(=O)CH₃ and -S(=O)CH₂CH₃.

Sulfone (sulfonyl): -S(=O)₂R, wherein R is a sulfone substituent, for example, a C₁₋₇ alkyl group, a C₃₋₂₀ heterocyclyl group, or a C₅₋₂₀ aryl group, preferably a C₁₋₇ alkyl group, including, for example, a fluorinated or perfluorinated C₁₋₇ alkyl group. Examples of sulfone groups include, but are not limited to, -S(=O)₂CH₃ (methanesulfonyl, mesyl), -S(=O)₂CF₃ (triflyl), -S(=O)₂CH₂CH₃ (esyl), -S(=O)₂C₄F₉ (nonaflyl), -S(=O)₂CH₂CF₃ (tresyl), -S(=O)₂CH₂CH₂NH₂ (tauryl), -S(=O)₂Ph (phenylsulfonyl, besyl), 4-methylphenylsulfonyl (tosyl), 4-chlorophenylsulfonyl (closyl), 4-bromophenylsulfonyl (brosyl), 4-nitrophenyl (nosyl), 2-naphthalenesulfonate (napsyl), and 5-dimethylamino-naphthalen-1-ylsulfonate (dansyl).

Sulfinic acid (sulfino): -S(=O)OH, -SO₂H.

Sulfonic acid (sulfo): -S(=O)₂OH, -SO₃H.

Sulfinate (sulfinic acid ester): -S(=O)OR; wherein R is a sulfinate substituent, for example, a C₁₋₇ alkyl group, a C₃₋₂₀ heterocyclyl group, or a C₅₋₂₀ aryl group, preferably a C₁₋₇alkyl group. Examples of sulfinate groups include, but are not limited to, -S(=O)OCH₃ (methoxysulfinyl; methyl sulfinate) and -S(=O)OCH₂CH₃ (ethoxysulfinyl; ethyl sulfinate).

Sulfonate (sulfonic acid ester): -S(=O)₂OR, wherein R is a sulfonate substituent, for example, a C₁₋₇ alkyl group, a C₃₋₂₀ heterocyclyl group, or a C₅₋₂₀ aryl group, preferably a C₁₋₇ alkyl group. Examples of sulfonate groups include, but are not limited to, -S(=O)₂OCH₃ (methoxysulfonyl; methyl sulfonate) and -S(=O)₂OCH₂CH₃ (ethoxysulfonyl; ethyl sulfonate).

Sulfinyloxy: -OS(=O)R, wherein R is a sulfinyloxy substituent, for example, a C₁₋₇ alkyl group, a C₃₋₂₀ heterocyclyl group, or a C₅₋₂₀ aryl group, preferably a C₁₋₇ alkyl group. Examples of sulfinyloxy groups include, but are not limited to, -OS(=O)CH₃ and -OS(=O)CH₂CH₃.

Sulfonyloxy: -OS(=O)₂R, wherein R is a sulfonyloxy substituent, for example, a C₁₋₇ alkyl group, a C₃₋₂₀ heterocyclyl group, or a C₅₋₂₀ aryl group, preferably a C₁₋₇ alkyl group. Examples of sulfonyloxy groups include, but are not limited to, -OS(=O)₂CH₃ (mesylate) and -OS(=O)₂CH₂CH₃ (esylate).

Sulfate: -OS(=O)₂OR; wherein R is a sulfate substituent, for example, a C₁₋₇ alkyl group, a C₃₋₂₀ heterocyclyl group, or a C₅₋₂₀ aryl group, preferably a C₁₋₇ alkyl group. Examples of sulfate groups include, but are not limited to, -OS(=O)₂OCH₃ and -SO(=O)₂OCH₂CH₃.

Sulfamyl (sulfamoyl; sulfinic acid amide; sulfinamide): -S(=O)NR¹R², wherein R¹ and R² are independently amino substituents, as defined for amino groups. Examples of sulfamyl groups include, but are not limited to, -S(=O) NH₂, -S(=O)NH(CH₃), -S(=O)N(CH₃)₂, -S(=O)NH(CH₂CH₃), -S(=O)N(CH₂CH₃)₂, and -S(=O)NHPh.

Sulfonamido (sulfinamoyl; sulfonic acid amide; sulfonamide): -S(=O)₂NR¹R², wherein R¹ and R² are independently amino substituents, as defined for amino groups. Examples of sulfonamido groups include, but are not limited to, -S(=O)₂NH₂, -S(=O)₂NH(CH₃), -S(=O)₂N(CH₃)₂, -S(=O)₂NH(CH₂CH₃), -S(=O)₂N(CH₂CH₃)₂, and -S(=O)₂NHPh.

Sulfamino: -NR¹S(=O)₂OH, wherein R¹ is an amino substituent, as defined for amino groups. Examples of sulfamino groups include, but are not limited to, -NHS(=O)₂OH and -N(CH3)S(=O)₂OH.

Sulfonamino: -NR¹S(=O)₂R, wherein R¹ is an amino substituent, as defined for amino groups, and R is a sulfonamino substituent, for example, a C₁₋₇ alkyl group, a C₃₋₂₀ heterocyclyl group, or a C₅₋₂₀ aryl group, preferably a C₁₋₇ alkyl group. Examples of sulfonamino groups include, but are not limited to, -NHS(=O)₂CH₃ and -N (CH₃) S (=O)₂C₆H₅.

Sulfinamino: -NR¹S(=O)R, wherein R¹ is an amino substituent, as defined for amino groups, and R is a sulfinamino substituent, for example, a C₁₋₇ alkyl group, a C₃₋₂₀ heterocyclyl group, or a C₅₋₂₀ aryl group, preferably a C₁₋₇ alkyl group. Examples of sulfinamino groups include, but are not limited to, -NHS(=O)CH₃ and -N(CH₃)S(=O)C₆H₅.

Phosphino (phosphine): -PR₂, wherein R is a phosphino substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphino groups include, but are not limited to, -PH₂, -P(CH₃)₂, -P(CH₂CH₃)₂, -P(t-Bu)₂, and -P(Ph)₂.

Phospho: -P(=O)₂.

Phosphinyl (phosphine oxide): -P(=O)R₂, wherein R is a phosphinyl substituent, for example, a C₁₋₇ alkyl group, a C₃₋₂₀ heterocyclyl group, or a C₅₋₂₀ aryl group, preferably a C₁₋₇ alkyl group or a C₅₋₂₀ aryl group. Examples of phosphinyl groups include, but are not limited to, -P(=O)(CH₃)₂, -P(=O)(CH₂CH₃)₂, -P(=O)(t-Bu)₂, and -P(=O)(Ph)₂.

Phosphonic acid (phosphono): -P(=O)(OH)₂.

Phosphonate (phosphono ester): -P(=O)(OR)₂, where R is a phosphonate substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphonate groups include, but are not limited to, -P(=O)(OCH₃)₂, -P(=O)(OCH₂CH₃)₂, -P(=O) (O-t-Bu)₂, and -P(=O)(OPh)₂.

Phosphoric acid (phosphonooxy): -OP(=O)(OH)₂.

Phosphate (phosphonooxy ester): -OP(=O)(OR)₂, where R is a phosphate substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphate groups include, but are not limited to, -OP(=O)(OCH₃)₂, -OP(=O)(OCH₂CH₃)₂, -OP(=O)(O-t-Bu)₂, and -OP(=O)(OPh)₂.

Phosphorous acid: -OP(OH)₂.

Phosphite: -OP(OR)₂, where R is a phosphite substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphite groups include, but are not limited to, -OP(OCH₃)₂, -OP(OCH₂CH₃)₂, -OP(O-t-Bu)₂, and -OP(OPh)₂,

Phosphoramidite: -OP(OR¹)-NR²₂, where R¹ and R² are phosphoramidite substituents, for example, -H, a (optionally substituted) C₁₋₇ alkyl group, a C₃₋₂₀ heterocyclyl group, or a C₅₋₂₀ aryl group, preferably - H, a C₁₋₇ alkyl group, or a C₅₋₂ aryl group. Examples of phosphoramidite groups include, but are not limited to, -OP(OCH₂CH₃)-N (CH₃)₂, -OP(OCH₂CH₃)-N(i-Pr)₂, and -OP(OCH₂CH₂CN)-N(i-Pr)₂.

Phosphoramidate:-OP(=O)(OR¹)-NR²₂, where R¹ and R² are phosphoramidate substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidate groups include, but are not limited to, -OP(=O)(OCH₂CH₃)-N (CH₃)₂, -OP(=O)(OCH₂CH₃)-N(i-Pr)₂, and -OP(=O)(OCH₂CH₂CN)-N(i-Pr)₂.

### Gene-based diseases

Gene-based diseases include, and are preferably, proliferative diseases, and also include Alzheimer's disease and bacterial, parasitic and viral infections. Any condition which may be treated by the regulation of gene expression may be treated using compounds of the fifth aspect of the invention.

### Proliferative Diseases

One of ordinary skill in the art is readily able to determine whether or not a candidate compound treats a proliferative condition for any particular cell type. For example, assays which may conveniently be used to assess the activity offered by a particular compound are described in the examples below.

The term "proliferative disease" pertains to an unwanted or uncontrolled cellular proliferation of excessive or abnormal cells which is undesired, such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours (e.g. histocytoma, glioma, astrocyoma, osteoma), cancers (e.g. lung cancer, small cell lung cancer, gastrointestinal cancer, bowel cancer, colon cancer, breast carinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma), leukemias, psoriasis, bone diseases, fibroproliferative disorders (e.g. of connective tissues), and atherosclerosis.

Any type of cell may be treated, including but not limited to, lung, gastrointestinal (including, e.g. bowel, colon), breast (mammary), ovarian, prostate, liver (hepatic), kidney (renal), bladder, pancreas, brain, and skin.

### Methods of Treatment

As described above, the present invention provide the use of a compound of the fifth aspect in a method of therapy. If the compounds of the fifth aspect include a PBD moiety, then this preferably comprises a N10-C11 imine bond, or has a N10 which is protected by a nitrogen protecting group (R¹⁰) which can be removed in vivo and the C11 substituent (R11) as OH. Also provided is a method of treatment, comprising administering to a subject in need of treatment atherapeutically-effective amount of a compound of of the fifth aspect, preferably in the form of a pharmaceutical composition, which is the third aspect of the present invention.

The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A compound may be administered alone or in combination with other treatments, either simultaneously or sequentially dependent upon the condition to be treated. Examples of treatments and therapies include, but are not limited to, chemotherapy (the administration of active agents, including, e.g. drugs; surgery; and radiation therapy. If the compound of formula of the fifth aspect comprises a PBD moiety which bears a carbamate-based nitrogen protecting group which may be removed in vivo, then the methods of treatment described in WO 00/12507 (ADEPT, GDEPT and PDT) may be used.

Pharmaceutical compositions according to the present invention, and for use in accordance with the present invention, may comprise, in addition to the active ingredient, i.e. a compound of formula of the fifth aspect, a pharmaceutically acceptable excipient, carrier, buffer, stabiliser or other materials well known to those skilled in the art. Such materials should be nontoxic and should not interfere with the efficacy of the active ingredient. The precise nature of the carrier or other material will depend on the route of administration, which may be oral, or by injection, e.g. cutaneous, subcutaneous, or intravenous.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

### Includes Other Forms

Unless otherwise specified, included in the above are the well known ionic, salt, solvate, and protected forms of these substituents. For example, a reference to carboxylic acid (-COOH) also includes the anionic (carboxylate) form (-COO-), a salt or solvate thereof, as well as conventional protected forms. Similarly, a reference to an amino group includes the protonated form (-N⁺HR¹R²), a salt or solvate of the amino group, for example, a hydrochloride salt, as well as conventional protected forms of an amino group. Similarly, a reference to a hydroxyl group also includes the anionic form (-O-), a salt or solvate thereof, as well as conventional protected forms.

### Isomers, Salts and Solvates

Certain compounds may exist in one or more particular geometric, optical, enantiomeric, diasteriomeric, epimeric, atropic, stereoisomeric, tautomeric, conformational, or anomeric forms, including but not limited to, cis- and transforms; E- and Z-forms; c-, t-, and r- forms; endo- and exo-forms; R-, S-, and meso-forms; D- and L-forms; d- and 1-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

Preferably if the compound of the fifth aspect comprise a PBD moiety then this moiety has the following stereochemistry at the C11 position:

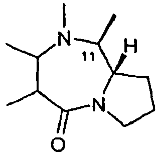

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to orthochlorophenyl is not to be construed as a reference to its structural isomer, meta-chlorophenyl. However, a reference to a class of structures may well include structurally isomeric forms falling within that class (e.g. C₁₋₇ alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hyroxyazo, and nitro/aci-nitro.

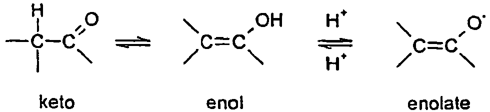

Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

Unless otherwise specified, a reference to a particular compound also includes ionic, salt, solvate, and protected forms of thereof, for example, as discussed below.

It may be convenient or desirable to prepare, purify, and/or handle a corresponding salt of the active compound, for example, a pharmaceutically-acceptable salt. Examples of pharmaceutically acceptable salts are discussed in Berge, et al., J. Pharm. Sci., 66, 1-19 (1977).

For example, if the compound is anionic, or has a functional group which may be anionic (e.g. -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of suitable organic cations include, but are not limited to, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g. -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

A particular salt form of interest can be formed from moieties of formula VI, where R¹⁰ and R¹¹ form an imine bond, by reacting said compound with a bisulphite salt to form a bisulphite derivative of the PBD. These compounds can be represented as: 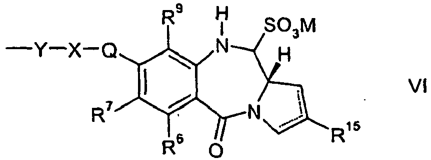 where M is a monovalent pharmaceutically acceptable cation, or if the compound is a dimer, the two M groups may together represent a divalent pharmaceutically acceptable cation, and the other groups are as previously defined.

It may be convenient or desirable to prepare, purify, and/or handle a corresponding solvate of the active compound. The term "solvate" is used herein in the conventional sense to refer to a complex of solute (e.g. active compound, salt of active compound) and solvent. If the solvent is water, the solvate may be conveniently referred to as a hydrate, for example, a monohydrate, a di-hydrate, a tri-hydrate, etc.

If the compounds of the fifth aspect comprise a PBD moiety then solvates of particular relevance are those where the solvent adds across the imine bond of the PBD moiety, which is illustrated below where the solvent is water or an alcohol (R^{A}OH, where R^{A} is an ether substituent as described above):

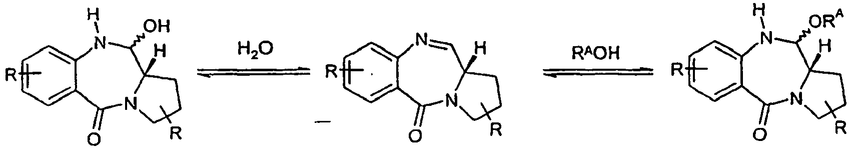

These forms can be called the carbinolamine and carbinolamine ether forms of the PBD. The balance of these equilibria depend on the conditions in which the compounds are found, as well as the nature of the moiety itself.

In general any nucleophilic solvent is capable of forming such solvates as illustrated above for hydroxylic solvents. Other nucleophilic solvents include thiols and amines.

These solvates may be isolated in solid form, for example, by lyophilisation.

### General synthetic routes

Compounds of formula I may be made by a variety of routes, some of which are discussed below.

### Modification of commercially available materials

Some compounds of formula 1 are commercially available:

HO₂C-A-B-NO₂ Formula 1

and can be readily modified to give compounds of formula I. The first step is protection of the carboxy group, for example as a methyl ester, using, for example EDCI, DMAP and MeOH in DMF. The nitro group can then be reduced to an amino group, for example using H₂ with a Pd/C catalyst in ethanol. The amino group can be protected, if necessary, for example by the use of Boc₂O in THF, and the carboxy group may be deprotected by hydrolysis, for example using NaOH.

### Heterocylic ring closures

If one of A and B is a heteroarylene group, then the compound of formula I can be synthesised from an appropriate precursor by means of a ring closure reaction.

For example, compounds of formula 2: 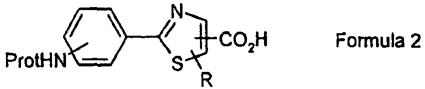 may be synthesised from a compound
of formula 3:

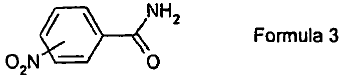

The compound of formula 3 can be converted into a compound of formula 4: 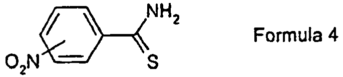 by using Lawessons regent, and this can then be ring closed to form a compounds of formula 5:

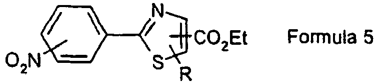

The ring closure may be accomplished by known methods, for example reaction with ethyl bromopyruvate or ethyl 2-chloroacetoaceatate. The nitro group may be converted to an amine group and protected in a similar manner to that discussed above, and the carboxy group may be deprotected also in a similar way to above.

### Suzuki coupling

Compounds of formula I may be synthesised by the coupling of the two C₅₋₆ arylene groups using Suzuki methodology. The groups -NHZ and -CO-Z' may be present on the C₅₋₆ arylene groups in their final form prior to coupling, or may be present as precursors (for example, a precursor to -NHZ is NO₂ - see above for conversion; a precursor of CO-Z' is -CHO, which can be converted by oxidation and optionally protected).

The coupling groups (e.g. Br and boronic acid/ester) may be either way round on A and B.

Suitable commercially available arylboronic acids/esters include:
Boronic acids and Esters

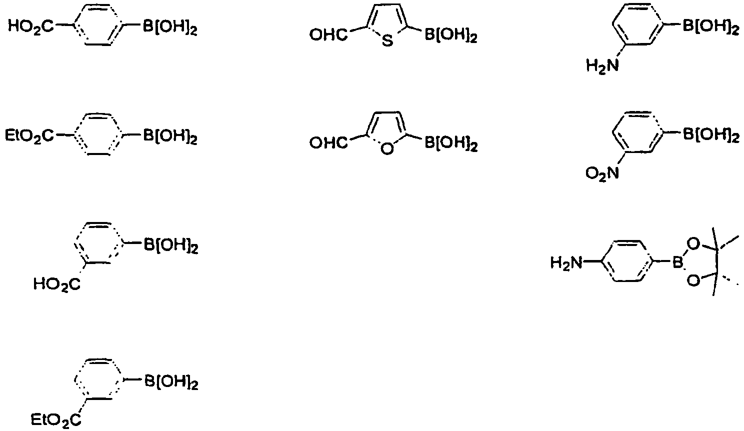

Suitable commercially available bromo compounds include:

| **1-Bromo-4-nitrobenzenes** | **1-Bromo-3-nitrobenzenes** | | **Pyridine Systems** |
|---|---|---|---|
| 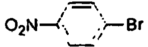 | 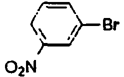 | 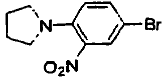 | 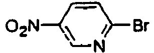 |
| 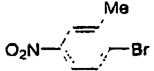 | 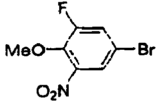 | 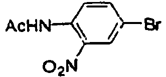 | 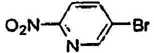 |
| 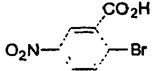 | | | |
| 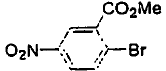 | 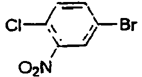 | 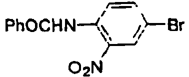 | 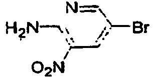 |
| 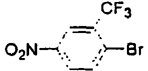 | 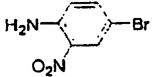 | 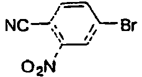 | 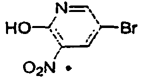 |
| 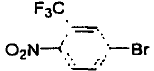 | 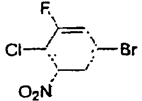 | 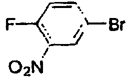 | 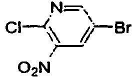 |
| 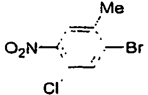 | 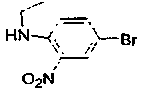 | 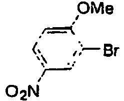 | 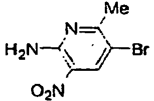 |
| 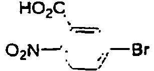 | 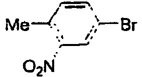 | | 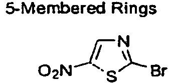 |
| 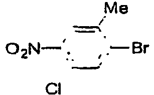 | 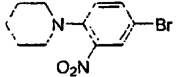 | | 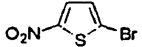 |

| **4-Bromobenzoic acids** | **3-Bromobenzoic acids** | | **Heterocyclic bromocarboxylic acids** |
|---|---|---|---|
| 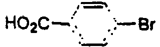 | 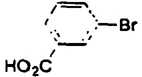 | 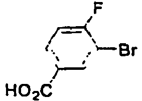 | 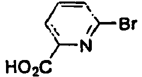 |
| 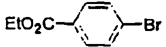 | 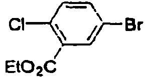 | 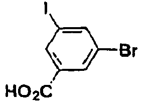 | 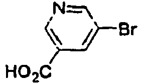 |
| 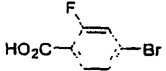 | 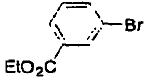 | 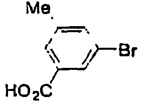 | 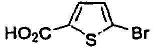 |
| 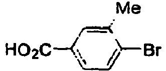 | 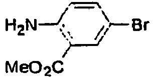 | 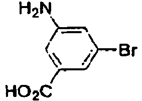 | 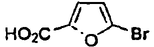 |
| | 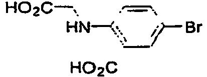 | 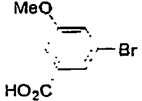 | 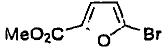 |
| | 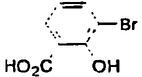 | | |
| | 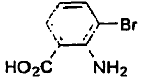 | | 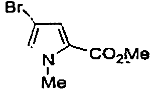 |
| | 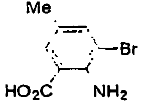 | | 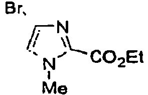 |

### Polyamido moieties

Polyamido moieties comprising a unit of formula II may be synthesised by reacting a compound of formula I with a compound having an amino or carboxy (or equivalent) terminating group. Typically, one end of compound I will be protected to prevent self-condensation. The other units described in the second aspect are well known, as are methods of amide bond formation. Typically, the carboxy group may be activated as an acid chloride group, or coupling initiators used, e.g. HOBt and EDCI.

### Pyrrolobenzodiazepine moieties

The synthesis of pyrrolobenzodiazepine moieties as described in the fifth aspect of the invention are described In WO 00/12506. Protection at the C11 position can be readily introduced.

### Compounds of formula IV

Compounds of formula IV may be synthesised by reacting a polyamido chain of formula 6:

Z"-(T)ₙ-Z' Formula 6

with a compound of formula 7:

Z"'-CO-(CH₂)_{q}-NR¹R² Formula 7

where Z"' is either OH or Cl, under amide bond forming conditions as described above. The compound of formula 6 may be formed as discussed above for polyamido moieties.

### Further preferences

The following preferences may apply to all aspects of the invention as described above, or may relate to a single aspect. The preferences may be combined together in any combination.

In the first aspect of the invention, it is preferred that if B is phenylene with -NH- β to the bond between A and B, then -A-CO- is not:

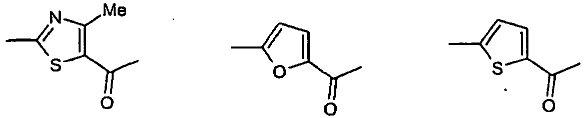

In the first aspect, it is also preferred that if -NH-B- is : 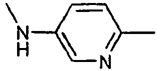 then -A-CO- is not:

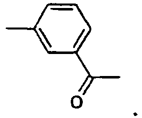

In the first aspect, it is further preferred that if B is phenylene, then A is not thiazolylene, furanylene or thiphenylene and if B is pyridylene, then A is not phenylene.

If Z' is a protected hydroxyl group, it is preferably and alkoxy group, and more preferably methoxy or ethoxy.

It is preferred that A and B are independently selected from phenylene, and arylene groups derived from C₅ heteroaryl groups having one or two heteroatoms, preferably at least one of which is nitrogen (i.e. pyrrole, oxazole, isoxazole, thiazole, isothiazole, imidazole and pyrazole). Of these, pyrrole, oxazole, thiazole and imidazole are preferred. The nitrogen atom of these groups may be substituted with a C₁₋₄ alkyl group, which is more preferably methyl.

The amino and carbonyl groups are preferably bound to A and B respectively at a position β- ory- to the bond between A and B (i.e. not adjacent to the bond between A and B).

In some embodiments, one of A and B is phenylene and the other of A and B is a C₅-heteroarylene group, preferably with one or two hetero ring atoms, one of which ring atoms is nitrogen.

Preferable substituents on A and B include, but are not limited to: C₁₋₄ alkyl (e.g. Me, CF₃), C₁₋₄ alkoxy (e.g. MeO, EtO), halo (e.g. Cl, F) and amino, preferably susbtituted by one or two C₁₋₄ alkyl groups.

Particularly preferred compounds of formula I are of formula la: 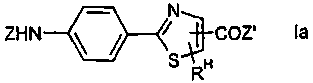 where R^{H} is selected from H and C₁₋₄ alkyl, and is preferably H or Me.

Particularly preferred units of formula II are of formula IIa: 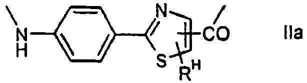 where R^{H} is selected from H and C₁₋₄ alkyl, and is preferably H or Me.

If compounds of the fifth aspect comprise a PBD moiety, then the following preferences are relevant:
R⁹ is preferably H.
R⁶ is preferably selected from H, OH, OR, SH, NH₂, nitro and halo, and is more preferably H or halo, and most preferably is H.
R⁷ is preferably independently selected from H, OR, SH, SR, NH₂, NHR, NRR', and halo, and more preferably independently selected from H and OR, where R is preferably selected from optionally substituted C₁₋₇ alkyl, C₃₋₁₀ heterocyclyl and C₅₋₁₀ aryl groups. Preferably R⁷ is OMe or H and most preferably OMe.
R¹⁰ is preferably BOC, Troc or alloc and is most preferably alloc.
R¹⁵ is preferably THP or a silyl oxygen protecting group (for example TBS) and is most preferably THP.

In other embodiments of the invention, R¹⁰ and R¹¹ together form a double bond between N10 and C11.

Q is preferably NH, O or a single bond and most preferably NH or O.

X is preferably a single bond or C₁₋₇ alkylene, more preferably a single bond or C₃ alkylene.

R³ is preferably H.

R² is preferably R, and is more preferably an optionally substituted C₅₋₂₀ aryl group. Most preferred is an optionally substituted phenyl group.

### Brief Description of Figure

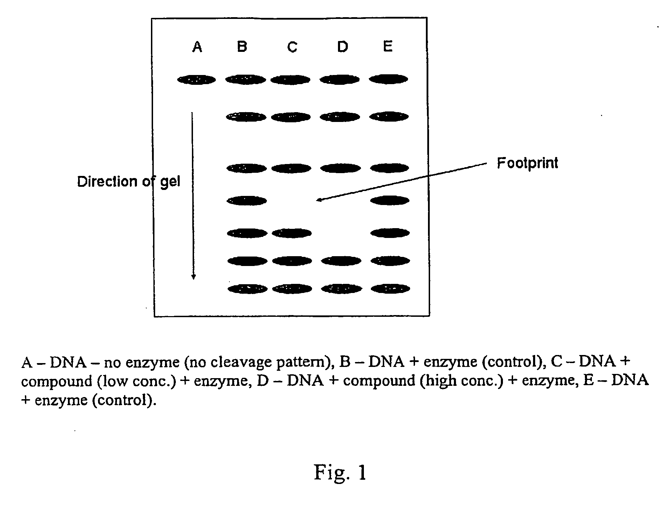

Figure 1 shows an illustrative example of an eletrophoresis gel in a DNA footprinting experiment (see Example 7).

### Example 1 - 2-(4-tert-Butyloxycarbonylaminophenyl)thiazole-4-carboxylic acid (4)

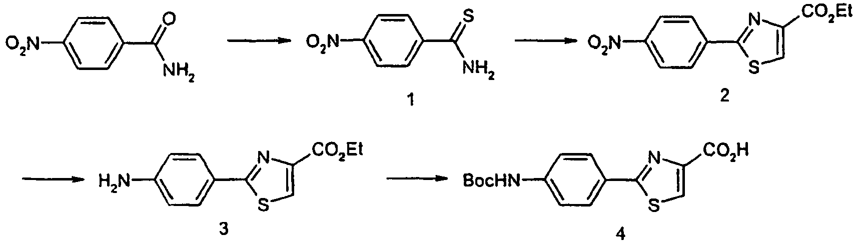

### (a) 4-Nitrothiobenzamide (1)

A suspension of 4-nitrobenzamide (5g, 30.1 mmol) in chlorobenzene (150mL) was stirred at 80°C and Lawessons reagent (7.3g, 18.1 mmol, 0.6equiv.) was added. The reaction mixture became orange/red in colour and all of the starting material dissolved. The solution was allowed to cool to room temperature and was stirred overnight. The precipitate formed was collected on a filter, washed with hexane then dried under vacuum. The crude product (6.0g) was recrystallised from ethanol/water to give the product **1** as gold coloured needles (3.81 g, 70%).
¹H NMR (d₆-DMSO) δ 10.20 (1 H, bs, N-H), 9.80 (1H, bs, N-H), 8.25 (2H, d, *J* = 8.9Hz, H-3,5), 8.02 (2H, d, *J* = 8.9Hz, H-2,6); ¹³C NMR (*d*₆-DMSO) δ 198.2, 148.5, 145.1, 128.4 (CH), 123.1 (CH).

### (b) Ethyl 2-(4-nitrophenyl)thiazole-4-carboxylate (2)

A suspension of the 4-nitrothiobenzamide (**1**)(3.5g, 19.2mmol) was stirred in ethanol (50mL) and ethyl bromopyruvate (3.75g, 19.2mmol, 1.0equiv.) was added. The mixture was heated at reflux for 4 hours then cooled and triethylamine (2.67mL, 19.2mmol, 1.0equiv.) added. The precipitate was collected on a filter, washed with water and dried under vacuum. The yield of white solid **2** was 4.01 g (75%).
¹H NMR (*d₆*-DMSO) δ 8.69 (1 H, s, H-5), 8.31 (2H, d, *J* = 9.0Hz, H-3',5'), 8.20 (2H, d, *J* = 9.0Hz, H-2',6'), 4.34 (2H, q, *J* = 7.1 Hz, CH₂), 1.33 (3H, t, *J*= 7.1 Hz, CH₃); ¹³C NMR (*d₆*-DMSO) δ 165.1, 160.4, 148.3, 147.5, 137.6, 131.1 (CH), 127.5 (CH), 124.5 (CH), 61.0 (CH₂), 14.1 (CH₃).

### (c) Ethyl 2-(4-aminophenyl)thiazole-4-carboxylate (3)

A solution of ethyl 2-(4-nitrophenyl)thiazole-4-carboxylate (2)(3.8g, 13.7mmol) and ammonium formate (5.17g, 82mmol) in ethanol (200mL) was stirred at room temperature. To this was added a suspension of 10%w/w palladium on charcoal (1.14g, 30%w/w) in ethanol (50mL). The reaction mixture was stirred for 36 hours at room temperature then filtered through celite. The celite pad was washed with hot ethanol (2 x 50mL). The combined filterates were concentrated to give a cream coloured crystalline solid 3 (3.65g). This was washed with water (3 x 50mL) and dried under vacuum. The yield was 2.252g, 66%).
¹H NMR (*d₆*-DMSO) δ 8.32 (1 H, s, H-5), 7.65 (2H, d, J = 8.6Hz, H-2',6'), 6.65 (2H, d, *J* = 8.6Hz, H-3',5'), 5.77 (2H, bs, N-H), 4.32 (2H, t, *J* = 7.2Hz, CH₂), 1.33 (3H, t, *J* = 7.2Hz, CH₃); ¹³C NMR (*d₆*-DMSO) δ 168.9, 160.9, 151.5, 146.4, 127.8 (CH), 126.4 (CH), 119.9, 113.6 (CH), 60.6 (CH₂), 14.2 (CH₃).

### (d) 2-(4-tert-Butyloxycarbonylaminophenyl)thiazole-4-carboxylic acid (4)

Ethyl 2-(4-aminophenyl)thiazole-4-carboxylate **(3)** (1 g, 4.0mmol) was dissolved in dry THF (25mL) and Boc anhydride (0.88g, 4.0mmol, 1.0equiv.) was added. The reaction mixture was heated at reflux under a nitrogen atmosphere for 18 hours. A further equivalent of Boc anhydride (0.88g, 4.0mmol) was then added and the mixture heated for a further 18 hours. The reaction mixture was cooled to room temperature and the solvent removed under vacuum. The residue was diluted with methanol (50mL) and then 1 M aqueous sodium hydroxide solution (50mL) was added. The reaction mixture was heated at reflux for 4 hours then cooled to room temperature and stirred overnight. The volume was reduced under vacuum and the aqueous solution acidified with 1 M hydrochloric acid (∼50mL) to pH 2-3. The resulting aqueous suspension was extracted with dichloromethane (4 x 50mL). The combined organic layers were dried over magnesium sulphate then concentrated under vacuum to give a pale yellow solid **4**, 1.27g (98%).
¹H NMR (*d₆*-DMSO) δ 13.10 (1H, bs, O-H), 9.70 (1H, bs, N-H), 8.42 (1H, s, H-5), 7.88 (2H, d, *J* = 8.7Hz, H-2',6'), 7.62 (2H, d, *J*= 8.7Hz, H-3',5'), 1.50 (9H, s, t-Bu CH₃); ¹³C NMR (*d₆*-DMSO) δ 167.3, 162.0, 152.5, 147.9, 141.9, 127.9 (CH), 127.1 (CH), 126.3, 1 18.2 (CH), 79.5, 28.0 (CH₃).

### Example 2 - 2-(4-tert-Butyloxycarbonylaminophenyl)thiazole-4-methyl-5-carboxylic acid (7)

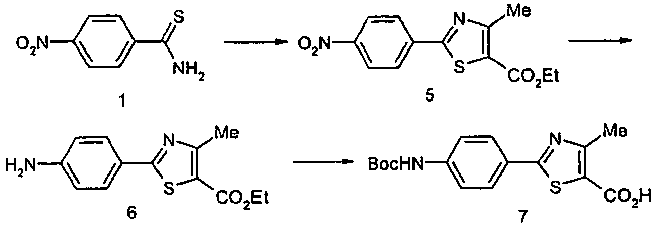

### (a) Ethyl 2-(4-nitrophenyl)thiazole-4-methyl-5-carboxylate (5)

This was made from 4-nitrothiobenzamide **(1)** by reacting with ethyl 2-chloroacetoacetate in a similar way to Example 1 (b).

### (b) Methyl 2-(4-aminophenyl)thiazole-4-methyl-5-carboxylate (6)

This was made from ethyl 2-(4-nitrophenyl)thiazole-4-methyl-5-carboxylate **(5)** using the method of Example 1 (c).

### (c) 2-(4-tert-Butyloxycatbonylaminophenyl)thiazole-4-methyl-5-carboxylic acid (7)

This was made from ethyl 2-(4-aminophenyl)thiazole-4-methyl-5-carboxylate **(6)** using the method of Example 1 (d).

### Example 3 - Ethyl 2-[4-({2-[4-(4-dimethylaminobutyrylamino)phenyllthiazole-4-carbonyl}amino)phenyl]thiazole-4-carboxylate (9)

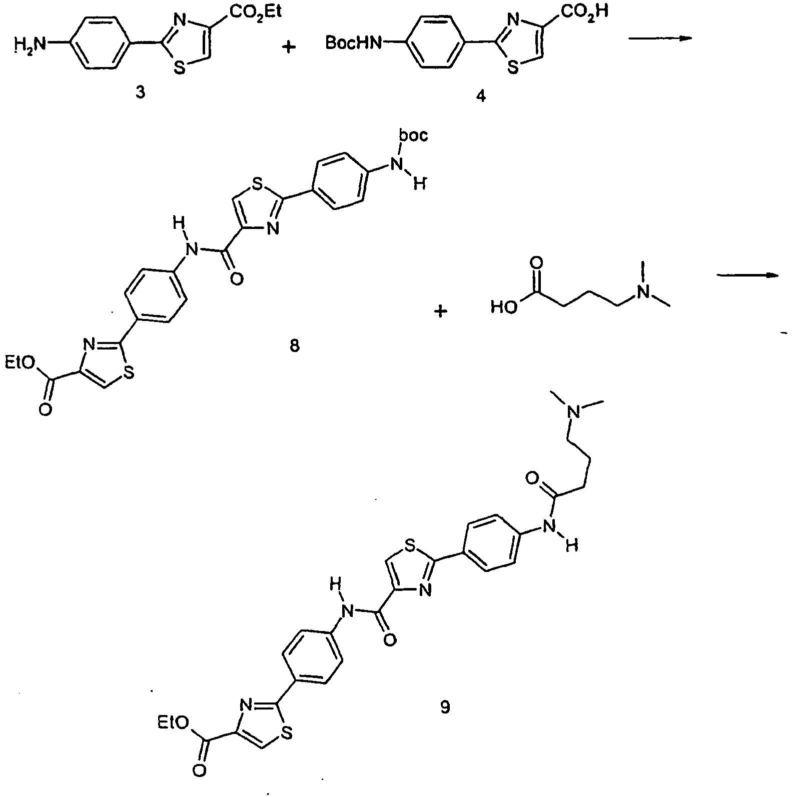

### (a) Ethyl 2-(4-{[2-(4-tert-butyloxycarbonylaminophenyl)thiazole-4-carbonyl]amino}phenyl)thiazole-4-carboxylate (8)

Ethyl 2-(4-aminophenyl)thiazole-4-carboxylate (3)(0.039g, 0.16mmol) and 2-(4-tert-butyloxycarbonylaminophenyl)thiazole-4-carboxylic acid (**4**)(0.050g, 0.16mmol) were dissolved in dry DMF (1 mL) and stirred under a nitrogen atmosphere. EDCI (0.060g, 0.16mmol, 2.0equiv.) and then DMAP (0.047g, 0.16mmol, 2.5equiv.) were added and the reaction mixture stirred at room temperature for 48 hours. The solution was diluted with ethyl acetate (10mL) and washed with 10%v/v hydrochloric acid (3 x 5mL) and then saturated sodium hydrogen carbonate solution (3 x 5mL). The organic layer was dried over magnesium sulphate then concentrated under vacuum to give an off white solid 8. The yield was 0.070g (81%).
¹H NMR (*d₆*-DMSO) δ 10.44 (1 H, bs, N-H), 9.73 (1 H, bs, Boc N-H), 8.56 (1 H, s, H-5), 8.46 (1 H, s, H-5), 8.09 (2H, d, *J* = 8.8Hz, phenyl-H), 8.08 (2H, d, *J*= 8.8Hz, phenyl-H), 8.02 (2H, d, *J* = 8.8Hz, phenyl-H), 7.66 (2H, d, *J*= 8.8Hz, phenyl-H), 4.36 (2H, q, *J* = 7.1 Hz, CH₂), 1.52 (9H, s, Boc CH₃), 1.35 (3H, t, *J* = 7.1 Hz, CH₃).

### (b) Ethyl 2-[4-({2-[4-(4-dimethylaminobutyrylamino)phenyl]thiazole-4-carbonyl}amino)phenyl]thiazole-4-carboxylate (9)

Ethyl 2-(4-{[2-(4-tert-butyloxycarbonylaminophenyl)thiazole-4-carbonyl]amino}phenyl)thiazole-4-carboxylate (8)(0.020g, 0.036mmol) was dissolved in a 4M solution of hydrogen chloride in dioxane (1 mL) with stirring. The reaction mixture was stirred for 1 hour under nitrogen, during which time a suspension formed. The solvent was removed under vacuum and the residue dried under vacuum. The residue and N,N-dimethylaminobutyric acid (0.016g, 0.12mmol, 3.3equiv) were dissolved in dry DMF (1 mL) and stirred under a nitrogen atmosphere. EDCI (0.060g, 0.16mmol, 2.0equiv.) and then DMAP (0.047g, 0.16mmol, 2.5equiv.) were added and the reaction mixture stirred at room temperature for 96 hours. The solution was diluted with ethyl acetate (15mL) and washed with saturated sodium hydrogen carbonate solution (3 x 5mL). The organic layer was dried over magnesium sulphate then concentrated under vacuum to give an off white solid 9. The yield was 0.021 g (93%).
¹H NMR (*d₆*-DMSO) δ 10.48 (1H, bs, N-H), 10.18 (1H, bs, N-H), 8.56 (2H, s, H-5), 8.20-7.98 (8H, m, phenyl-H), 4.36 (2H, t, *J* = 7.1 Hz, CH₂), 2.45-1.53 (6H, m, butyryl C-H), 2.17 (3H, s, N-CH₃), 2.12 (3H, s, N-CH₃), 1.36 (3H, t, *J*= 7.1 Hz, CH₃).

### Example 4 - 5-(4-tert-Butoxycarbonylaminophenyl)-furan-2-carboxylic acid (12)

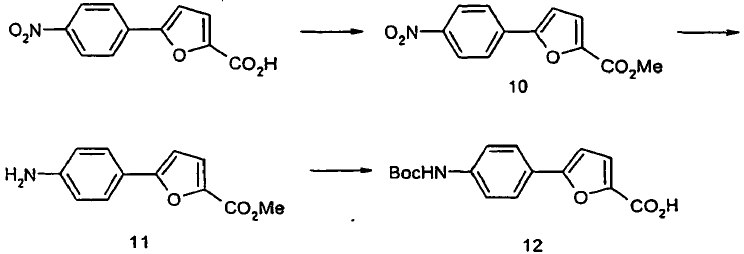

### (a) Methyl 5-(4-nitrophenyl)-2-furoate (10)

A suspension of 5-(4-nitrophenyl)-2-furoic acid (4.9g, 21.0mmol) was suspended in dry dichloromethane (50mL) and oxalyl chloride (2.998g, 23.6mmol, 1.1equiv.) was added with stirring. After 5 minutes DMF (2 drops) was added and the flask fitted with a calcium chloride drying tube. The reaction mixture was stirred overnight during which time a homogeneous solution formed. A solution of triethylamine (4.768g, 46.2mmol, 2.2 equiv.) in dry methanol (20mL) was added dropwise to the acid chloride over 30 minutes. The reaction mixture was stirred for a further two hours then the concentrated under vacuum. The residue was taken up in ethyl acetate (200mL) and washed with 1 M hydrochloric acid (3 x 50mL) and saturated sodium hydrogen carbonate solution (3 x 50mL). The organic layer was dried over magnesium sulphate then concentrated under vacuum to a cream coloured solid 10, 4.972g (96%).
¹H NMR (*d*_{*6*-}DMSO) δ 8.32 (2H, d, *J*= 9.0Hz, H-3',5'), 8.06 (2H, d, *J* = 9.0Hz, H-2',6'), 7.48 (2H, s, H-3,4), 3.88 (3H, s, OCH₃); ¹³C NMR (*d₆*-DMSO) δ 158.1, 154.2, 147.0, 144.5, 135.2, 134.5, 125.3 (CH), 124.5 (CH), 120.5 (CH), 111.6 (CH), 52.0 (CH₃); LCMS R_{T} = 3.50 min, (M⁺+1) = 248.

### (b) Methyl 5-(4-aminophenyl)-2-furoate (11)

A solution/suspension of methyl 5-(4-nitrophenyl)-2-furoate (10) (5.083g, 20.6mmol) in ethyl acetate (240mL) was added a suspension of 10% palladium on charcoal (0.5g, 10%equiv.) in ethyl acetate (10mL). The mixture was agitated under a hydrogen atmosphere (30psi) for 4 hours, then filtered through a celite pad. The celite was washed with ethyl acetate (2 x 50mL) and the combined filtrates concentrated under vacuum to give a pale yellow solid 11, 3.905g (87%).
¹H NMR (*d₆*-DMSO) δ 7.50 (2H, d, *J*= 8.6Hz, H-2',6'), 7.34 (1H, d, *J* = 3.4Hz, H-3), 6.79 (1H, d, *J* 3.6Hz, H-4), 6.66 (2H, d, *J*= 8.6Hz, H-3',5'), 5.59 (2H, bs, N-H), 3.82 (3H, s, OCH₃); ¹³C NMR (*d₆*-DMSO) δ 158.7, 158.4, 150.0, 141.1, 125.9 (CH), 120.9 (CH), 116.5, 113.7 (CH), 104.0 (CH), 51.5 (CH₃); LCMS R_{T} = 2.73 min, (M⁺+1) = 218.

### (c) 5-(4-tert-Butoxycarbonylaminophenyl)-furan-2-carboxylic acid (12)

To the methyl 5-(4-nitrophenyl)-2-furoate (**11**)(1.5g, 6.1 mmol) dissolved in ethyl acetate (100mL) was added a suspension of 10% palladium on charcoal (0.3g, 20%equiv.) in ethyl acetate (20mL). The mixture was shaken under a hydrogen atmosphere (20psi) for 4 hours, then filtered through a celite pad. The celite was washed with ethyl acetate (2 x 20mL) and the combined filterates concentrated under vacuum. The residue was dissolved in dry THF (40mL) and Boc anhydride (1.323g, 6.1 mmol, 1.0equiv.) was added. The mixture was stirred at room temperature then heated at reflux for 6 hours. The solvent was removed under vacuum and the residue dissolved in ethyl acetate (100mL). The solution was washed with 1 M hydrochloric acid (3 x 50mL), then water (1 x 50mL). The organic layer was dried over magnesium sulphate then concentrated under vacuum to give a yellow solid. This was dissolved/suspended in methanol (50mL) and 1 M sodium hydroxide (1 00mL) was added, then the mixture was heated at reflux for 4 hours then cooled and the methanol removed under vacuum. The solution was acidified with 1 M hydrochloric acid (-100mL) then extracted with ethyl acetate (3 x 100mL). The combined organic extracts were dried over magnesium sulphate and then concentrated under vacuum to give a yellow solid **12**, 1.454g, (76%).
¹H NMR (*d₆*-DMSO) δ 9.57 (1 H, bs, N-H), 7.69 (2H, d, *J*= 8.7Hz, H-2',6'), 7.56 (2H, d, *J* = 8.7Hz, H-3',5'), 7.28 (1 H, d, *J*= 3.6Hz, H-3), 6.98 (1 H, d, *J* = 3.6Hz, H-4), 1.48 (9H, s, Boc-CH₃); ¹³C NMR (*d₆*-DMSO) δ 159.2, 156.5, 152.6, 143.4, 140.2, 125 (CH), 123.0, 120.0 (CH), 118.2 (CH), 106.4 (CH), 79.3, 28.0 (CH₃).

### Example 5 - Methyl 4'-[(4-{[4-(4-butoxycarbonylamino)-1-methyl-1H-pyrrole-2-carbonyl]amino}-1-methyl-1H-pyrrole-2-carbonyl)amino]phenyl-2-furoate (13)

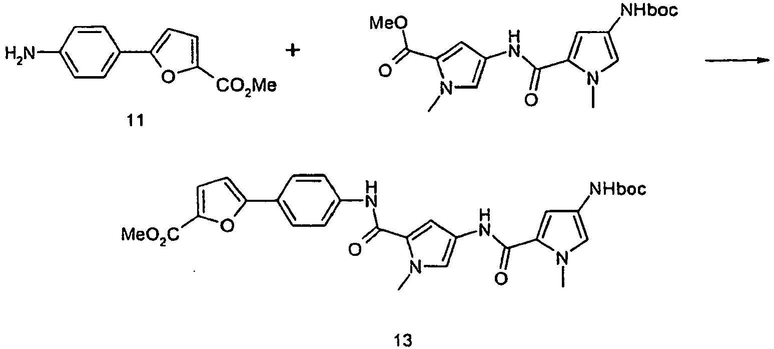

A solution of methyl 5-(4-aminophenyl)-2-furoate (11)(0.085g, 0.39mmol) and methyl 4-[(4-tert-butoxycarbonylamino-1-methyl-1H-pyrrole-2-carbonyl)-amino]-1-methyl-1H-pyrrole-2-carboxylate (0.15g, 0.41 mmol, 1.05equiv.) in dry DMF (2mL) was added a suspension of EDCl (0.159g, 0.83mmol, 2.0equiv.)in dry DMF (1 mL) and dry dichloromethane (1mL) followed by DMAP (0.126g, 1.03mmol, 2.5equiv.) dissolved in dry DMF (0.5mL). The reaction mixture was stirred at room temperature for 8 days then concentrated under vacuum to a volume of ∼1 mL. The residue was taken up in ethyl acetate (20mL) and washed with 10%v/v hydrochloric acid (3 x 10mL), then saturated sodium hydrogen carbonate solution (3 x 1 0mL). The organic fraction was dried over magnesium sulphate then concentrated under vacuum to give a yellow oil 13, which was purified by column chromatography (silica gel, chloroform/methanol 1/99).
¹H NMR (*d₆*-DMSO) δ 10.05 (1 H, bs, N-H), 9.90 (1 H, bs, N-H), 9.10 (1 H, bs, N-H), 7.90 (2H, d, *J* = 8.8Hz, H-2',6'), 7.78 (2H, d, *J* = 8.8Hz, H-3',5'), 7.43 (1 H, d, *J* = 3.6Hz, py-H), 7.31 (1 H, d, *J* = 1.5Hz, py-H), 7.21 (1 H, d, *J* 1.4Hz, py-H), 7.09 (1 H, d, *J*= 3.6Hz, py-H), 6.92 (1 H, bs, N-H), 6.87 (1 H, bs, N-H), 3.88 (3H, s, OCH₃), 3.86 (3H, s, OCH₃), 3.84 (3H, s, OCH₃), 1.48 (9H, s, Boc-CH₃).

### Example 6 - Methyl 4'-[(4-{[4-(4-butoxycarbonylamino)-1-methyl-1H-pyrrole-2-carbonyl]amino}-1-methyl-1H-pyrrole-2-carbonyl)amino]biphenyl-3-carboxylate (17)

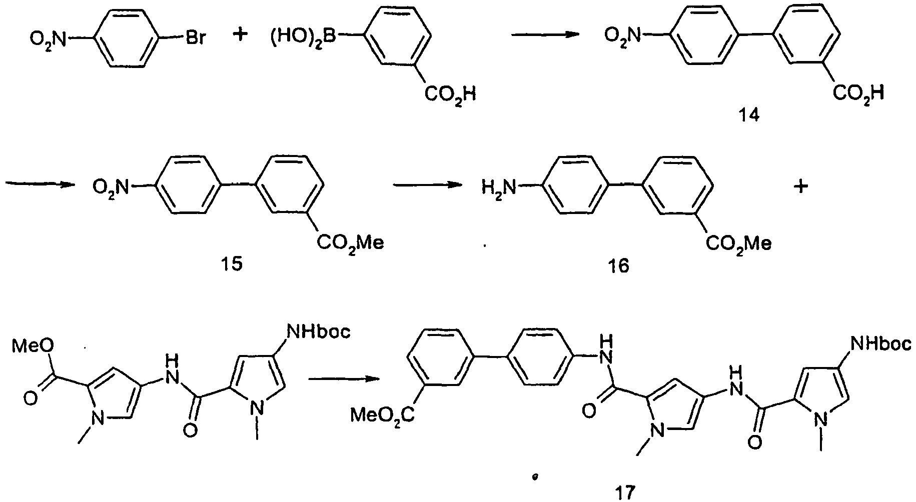

### (a) 3-(4-Nitrophenyl)benzoic acid (14)

1-Bromo-4-nitrobenzene (1.95g, 9.6mmol) and 3-carboxybenzeneboronic acid (1.8g, 10.8mmol, 1.1equiv.) were dissolved in a mixture of toluene (40mL), ethanol (40mL) and water (5mL) and potassium carbonate (4.1 g, 29.3mmol, 3.0equiv.) was added. The flask was purged with nitrogen gas then palladium tetrakis(triphenylphosphine) (0.2g) was added and the mixture heated at reflux for 48 hours. The reaction mixture was cooled to room temperature and diluted with ethyl acetate (100mL) and extracted with water (3 x 50mL). The aqueous extracts were combined and washed with dichloromethane (3 x 50mL). The aqueous fraction was acidified (pH 1-2) with concentrated hydrochloric acid to give an off white precipitate, which was collected on a filter and dried under vacuum to give a white solid **14**, 2.34g, (100%). ¹H NM R (d₆-DMSO) δ 13.20 (1 H, bs, OH), 8.36-8.29 (3H, m, Ar-H), 8.07-8.01 (4H, m, Ar-H), 7.69 (1 H, d, *J* = 7.8Hz, H-6); ¹³C NMR (*d₆*-DMSO) δ 166.9, 146.9, 145.6, 138.2, 131.7 (CH), 131.6, 129.6 (CH), 128.0 (CH), 127.8 (CH), 124.1; LCMS R_{T} = 3.28 min, (M⁻-1) = 242.

### (b) Methyl 3-(4-nitrophenyl)benzoate (15)

A suspension of 3-(4-nitrophenyl)benzoic acid (14) (2g, 8.2mmol) in dry dichloromethane (50mL) and oxalyl chloride (1.15g, 9.0mmol, 1.1 equiv.) was added. After 5 minutes DMF (2 drops) was added and the flask fitted with a calcium chloride drying tube. The reaction mixture was stirred overnight during which time a homogeneous solution formed. A solution of triethylamine (1.815g, 17.9mmol, 2.2 equiv.) in dry methanol (10mL) was added dropwise to the acid chloride over 20 minutes. The reaction mixture was stirred for a further two hours then the concentrated under vacuum. The residue was taken up in ethyl acetate (150mL) and washed with 1 M hydrochloric acid (3 x 50mL) and saturated sodium hydrogen carbonate solution (3 x 50mL). The organic layer was dried over magnesium sulphate then concentrated under vacuum to a cream coloured solid **15**, 1.966g (88%).
¹H NMR (*d₆*-DMSO) δ 8.33 (2H, d, *J*= 8.9Hz, H-3',5'), 8.27 (1H, m, Ar-H), 8.13-8.04 (2H, m, Ar-H), 8.01 (2H, d, *J*= 8.9Hz, H-2',6'), 7.71 (1 H, dd, *J*= 7.8Hz, H-5), 3.92 (3H, s, OCH₃); ¹³C NMR (*d₆*-DMSO) δ 165.9, 147.0, 145.4, 138.3, 132.0 (CH), 130.6, 129.8 (CH), 129.5 (CH), 128.1 (CH), 127.6 (CH), 124.1 (CH), 52.3 (CH₃); LCMS R_{T} = 2.77 min, (M⁺+1) = 228.

### (c) Methyl 3-(4-aminophenyl)benzoate (16)

A solution of methyl 3-(4-aminophenyl)benzoate (15)(1.85g, 6.8mmol) in ethyl acetate (100mL) was added a suspension of 10% palladium on charcoal (0.185g, 10%equiv.) in ethyl acetate (10mL). The mixture was agitated under a hydrogen atmosphere (30psi) for 4 hours, then filtered through a celite pad. The celite was washed with ethyl acetate (3 x 50mL) and the combined filtrates concentrated under vacuum to give a pale yellow solid **16**, 1.663g (100%).
¹H NMR (*d₆*-DMSO) δ 8.09 (1H, dd, *J* = 1.7Hz, H-2), 7.85-7.80 (2H, m, H-4,6), 7.54 (1 H, dd, *J* = 7.8Hz, H-5), 7.41 (2H, d, *J* = 8.5Hz, H-2',6'), 6.68 (2H, d, *J*= 8.5Hz, H-3',5'), 3.89 (3H, s, OCH₃); ¹³C NMR (*d₆*-DMSO) δ 166.4, 148.9, 141.2, 130.1 (CH), 129.2 (CH), 127.3 (CH), 126.2 (CH), 126.0, 125.6 (CH), 114.3 (CH), 52.1 (CH₃).

### (d) Methy/4'-[(4-{[4-(4-butoxycarbony/amino)-1-methy/-1H-pyrrole-2-carbonyl]amino}-1-methy/-1H-pyrrole-2-carbonyl)amino]biphenyl-3-carboxylate (17)

A solution of methyl 3-(4-aminophenyl)benzoate (**16**) (0.095g, 0.39mmol) and methyl 4-[(4-tert-butoxycarbonylamino-1-methyl-1H-pyrrole-2-carbonyl)-amino]-1-methyl-1H-pyrrole-2-carboxylate (0.15g, 0.41 mmol, 1.05equiv.) in dry DMF (2mL) was added a suspension of EDCI (0.159g, 0.83mmol, 2.0equiv.) in dry DMF (1 mL) and dry dichloromethane (1mL) followed by DMAP (0.126g, 1.03mmol, 2.5equiv.) dissolved in dry DMF (0.5mL). The reaction mixture was stirred at room temperature for 8 days then concentrated under vacuum to a volume of ∼1 mL. The residue was taken up in ethyl acetate (20mL) and washed with 10%v/v hydrochloric acid (3 x 10mL), then saturated sodium hydrogen carbonate solution (3 x 1 0mL). The organic fraction was dried over magnesium sulphate then concentrated undervacuum to give a yellow oil 17, which was purified by column chromatography (silica gel, chloroform/methanol 1/99).
¹H NMR (CDCl₃) δ 8.28 (1 H, dd, *J* = 1.6Hz, Ar-H), 8.00 (1H, dd, *J* = 1.3, 7.8Hz, Ar-H), 7.78 (1 H, m, Ar-H), 7.69-7.60 (6H, m, Ar-H), 7.51 (1H, dd, *J*= 1.7Hz, H-5 (biphenyl)), 7.45 (1H, bs, N-H), 7.15 (1H, d, *J*= 1.7Hz, py-H), 6.82 (1H, d, *J* = 1.8Hz, py-H), 6.61 (1H, bs, N-H), 6.22 (1H, bs, N-H), 3.98 (3H, s, CH₃), 3.96 (3H, s, CH₃), 3.93 (3H, s, CH₃), 1.55 (9H, s, Boc-CH₃).

### Example 7 - Methyl 3-(5-aminopyridin-2-yl)benzoate (20)

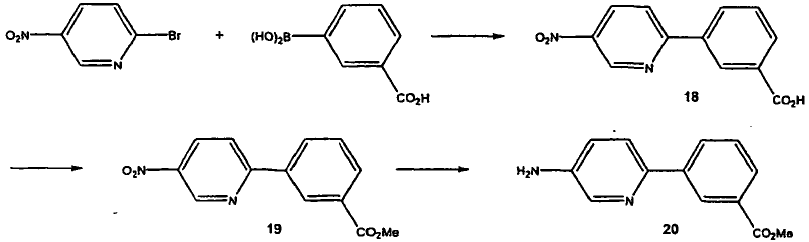

### (a) 3-(5-Nitropyridin-2-yl)benzoic acid (18)

2-Bromo-5-nitropyridine(1.96g, 9.6mmol) and 3-carboxybenzeneboronic acid (1.8g, 10.8mmol, 1.1equiv.) were dissolved in a mixture of toluene (40mL), ethanol (40mL) and water (5mL) and potassium carbonate (4.1g, 29.3mmol, 3.0equiv.) was added. The flask was purged with nitrogen gas then palladium tetrakis(triphenylphosphine) (0.2g) was added and the mixture heated at reflux for 48 hours. The reaction mixture was cooled to room temperature and diluted with ethyl acetate (100mL) and extracted with water (3 x 50mL). The aqueous extracts were combined and washed with dichloromethane (3 x 50mL). The aqueous fraction was acidified (pH 1-2) with concentrated hydrochloric acid to give an off white precipitate, which was collected on a filter and dried under vacuum to give a white solid 18, 2.132g (91 %). ¹H-NMR (*d₆*-DMSO) δ 13.21 (1 H, bs, CO₂H), 9.46 (1 H, d, *J* = 2.7Hz, H-6'), 8.75 (1 H, dd, *J* = 1.6Hz, H-2), 8.66 (1 H, dd, *J =* 2.7, 8.8Hz, H-4'), 8.42 (1 H, ddd, *J* = 1.2, 1.9, 7.9Hz, H-6), 8.33 (1 H, dd, *J* = 8.8Hz, H-3'), 8.10 (1 H, m, H-4), 7.69 (1H, dd, *J*= 7.8Hz, H-5); ¹³C NMR (*d₆*-DMSO) δ 166.8, 159.9, 144.9 (CH), 143.3, 136.9, 132.8 (CH), 131.7, 131.6 (CH), 131.3 (CH), 129.5 (CH), 128.2 (CH), 120.8 (CH); LCMS R_{T} = 3.00 min, (M⁻-1) = 243.

### (b) Methyl 3-(5-nitropyridin-2-yl)benzoate (19)

A suspension of 3-(4-nitrophenyl)benzoic acid (2g, 8.2mmol) in dry dichloromethane (50mL) and oxalyl chloride (1.15g, 9.0mmol, 1.1equiv.) was added. After 5 minutes DMF (2 drops) was added and the flask fitted with a calcium chloride drying tube. The reaction mixture was stirred overnight during which time a homogeneous solution formed. A solution of triethylamine (1.815g, 17.9mmol, 2.2 equiv.) in dry methanol (10mL) was added dropwise to the acid chloride over 20 minutes. The reaction mixture was stirred for a further two hours then the concentrated under vacuum. The residue was taken up in ethyl acetate (150mL) and washed with 1 M hydrochloric acid (3 x 50mL) and saturated sodium hydrogen carbonate solution (3 x 50mL). The organic layer was dried over magnesium sulphate then concentrated under vacuum to a white solid 19, 1.936g (86%).
¹H NMR (*d₆*-DMSO) δ 9.47 (1H, d, *J*=2.4Hz, H-6'), 8.77 (1H, dd, *J*= 1.7Hz, H-2), 8.67 (1H, dd, *J*= 2.7, 8.8Hz, H-4'), 8.46 (1 H, m, H-6), 8.35 (1H, d, *J*= 8.6Hz, H-3'), 8.11 (1 H, m, H-4), 7.72 (1 H, dd, *J* = 7.8Hz, H-5), 3.91 (3H, s, OCH₃); ¹³C NMR (*d₆*-DMSO) δ 165.8, 159.7, 144.9 (CH), 143.4, 137.0, 132.9 (CH), 132.0 (CH), 131.1 (CH), 130.5, 129.7 (CH), 128.0 (CH), 120.9 (CH), 52.3 (CH₃); LCMS R_{T} = 3.58 min, (M⁺+1) = 259.

### (c) Methyl 3-(5-aminopyridin-2-yl)benzoate (20)

A solution of methyl 3-(4-aminophenyl)benzoate (1.80g, 6.6mmol) in ethyl acetate (100mL) was added a suspension of 10% palladium on charcoal (0.185g, 10%equiv.) in ethyl acetate (10mL). The mixture was agitated under a hydrogen atmosphere (30psi) for 4 hours, then filtered through a celite pad. The celite was washed with ethyl acetate (3 x 50mL) and the combined filtrates concentrated under vacuum to give an oil which solidified on cooling, 1.598g (100%) of 20.
¹H-NMR (*d₆*-DMSO) δ 8.56 (1 H, dd, *J* = 1.7Hz, H-2), 8.15 (1 H, m, H-6), 8.06 (1 H, d, *J* 2.6Hz, H-6'), 7.85 (1 H, m, H-4), 7.70 (1 H, d, *J* = 8.5Hz, H-3'), 7.53 (1 H, dd, *J* = 7.8Hz, H-5), 7.02 (1 H, dd, *J* = 2.8, 8.5Hz, H-4'), 5.59 (2H, bs, NH₂), 3.88 (3H, s, OCH₃); ¹³C NMR (*d₆*-DMSO) δ 166.4, 144.6, 142.2, 139.8, 136.1 (CH), 129.9, 129.3 (CH), 129.0 (CH), 127.4 (CH), 125.5 (CH), 120.4 (CH), 52.1 (CH₃); LCMS R_{T} = 1.88 min, (M⁺+1) = 229.

### Example 8 - 5-(3-tert-Butoxycarbonylaminophenyl)-furan-2-carboxylic acid (23)

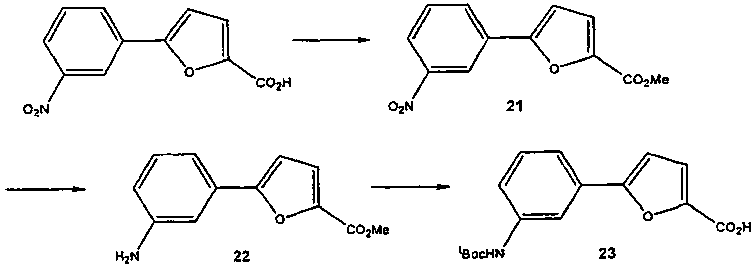

### (a) Methyl 5-(3-nitrophenyl)-2-furoate (21)

A suspension of 5-(3-nitrophenyl)-2-furoic acid (5.0g, 21.4mmol) was suspended in dry dichloromethane (50mL) and oxalyl chloride (2.998g, 23.6mmol, 1.1 equiv.) was added with stirring. After 5 minutes DMF (2 drops) was added and the flask fitted with a calcium chloride drying tube. The reaction mixture was stirred overnight during which time a homogeneous solution formed. A solution of triethylamine (4.768g, 46.2mmol, 2.2 equiv.) in dry methanol (20mL) was added dropwise to the acid chloride over 30 minutes. The reaction mixture was stirred for a further two hours then the concentrated under vacuum. The residue was taken up in ethyl acetate (200mL) and washed with 1 M hydrochloric acid (3 x 50mL) and saturated sodium hydrogen carbonate solution (3 x 50mL). The organic layer was dried over magnesium sulphate then concentrated under vacuum to an off white solid 21, 5.170g (98%).
¹H NMR (*d₆*-DMSO) δ 8.53 (1 H, dd, *J* = 1.9Hz, H-2'), 8.24 (2H, m, H-4',6'), 7.78 (1 H, dd, *J* = 8.0Hz, H-5'), 7.46 (2H, m, H-3,4), 3.86 (3H, s, OCH₃); ¹³C NMR (*d₆*-DMSO) δ 158.1, 154.1, 148.4, 143.8, 130.8 (CH), 130.6 (CH), 130.3, 123.4 (CH), 120.4 (CH), 118.7 (CH), 110.3 (CH), 52.0 (CH₃); LCMS R_{T} = 3.53 min, (M⁺+1) = 248.

### (b) Methyl 5-(3-aminophenyl)-2-furoate (22)

A solution/suspension of methyl 5-(3-nitrophenyl)-2-furoate (5.047g, 20.4mmol) in ethyl acetate (240mL) was added a suspension of 10% palladium on charcoal (0.5g, 10%equiv.) in ethyl acetate (10mL). The mixture was agitated under a hydrogen atmosphere (30psi) for 4 hours, then filtered through a celite pad. The celite was washed with ethyl acetate (2 x 50mL) and the combined filtrates concentrated under vacuum to give a pale yellow solid **22**, 4.419g (100%).
¹H NMR (*d₆*-DMSO) δ 7.37 (1 H, d, J = 3.7Hz, H-3), 7.11 (1H, dd, J = 7.8Hz, H-5'), 7.03 (1H, dd, J = 1.9Hz, H-2'), 6.97 (1 H, d, J = 3.7Hz, H-4), 6.95 (1H, m, H-6'), 6.60 (1 H, m, H-4'), 5.31 (2H, s, NH₂), 3.83 (3H, s, OCH₃); ¹³C NM R (*d₆*-DMSO) δ 158.3, 157.7, 149.2, 142.4, 129.5 (CH), 129.4, 120.5 (CH), 114.8 (CH), 112.4 (CH), 109.2 (CH), 107.2 (CH), 51.7 (CH₃); LCMS R_{T} = 2.58 min, (M⁺+1) = 218.

### (c) 5-(3-tert-Butoxycarbonylaminophenyl)-furan-2-carboxylic acid (23)

To the methyl 5-(3-nitrophenyl)-2-furoate (1.5g, 6.1 mmol) dissolved in ethyl acetate (100mL) was added a suspension of 10% palladium on charcoal (0.3g, 20%equiv.) in ethyl acetate (20mL). The mixture was shaken under a hydrogen atmosphere (20psi) for 3 hours, then filtered through a celite pad. The celite was washed with ethyl acetate (2 x 20mL) and the combined filterates concentrated under vacuum. The residue was dissolved in dry THF (40mL) and Boc anhydride (1.323g, 6.1 mmol, 1.0equiv.) was added. The mixture was stirred at room temperature then heated at reflux for 6 hours. The solvent was removed under vacuum and the residue dissolved in ethyl acetate (100mL). The solution was washed with 1 M hydrochloric acid (3 x 50mL), then water (1 x 50mL). The organic layer was dried over magnesium sulphate then concentrated under vacuum to give an oil (Boc protected amine with excess Boc anhydride). This was dissolved in methanol (40mL) and 1 M sodium hydroxide (100mL) was added, then the mixture was heated at 60°C for 6 hours then cooled and the methanol removed under vacuum. The solution was acidified with 1 M hydrochloric acid to pH -4. The resulting suspension was extracted with dichloromethane (4 x 50mL). The combined organic extracts were dried over magnesium sulphate and then concentrated under vacuum to give an off white solid, 1.572g, (82%). ¹H NMR (*d₆*-DMSO) δ 13.09 (1 H, s, OH), 9.50 (1 H, bs, NH), 8.00 (1 H, m, H-2'), 7.42 (1 H, m, H-4'/6'), 7.40 (1 H, m, H-4'/6'), 7.34 (1 H, m, H-5'), 7.30 (1 H, d, *J* = 3.6Hz, H-3), 7.03 (1 H, d, *J* = 3.6Hz, H-4), 1.49 (9H, s, [CH₃]₃); ¹³C NMR (*d₆*-DMSO) δ 159.2, 156.2, 152.7, 144.1, 140.2, 129.5, 129.4 (CH), 119.8 (CH), 118.6 (CH), 118.5 (CH), 113.5 (CH), 107.8 (CH), 79.2, 28.1 (CH₃).

### Example 9 - Ethyl 5-(4-aminophenyl)thiophene-2-carboxylate (25)

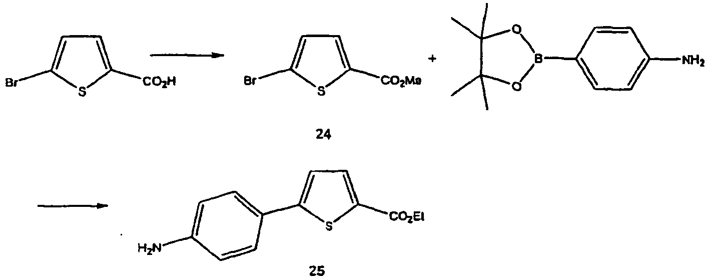

### (a) Methyl 5-bromothiophene-2-carboxylate (24)

A suspension of 5-bromothiophene-2-carboxylic acid (1.0g, 4.8mmol) was suspended in dry dichloromethane (10mL) and oxalyl chloride (0.675g, 5.3mmol, 1.1 equiv.) was added with stirring. After 5 minutes DMF (2 drops) was added and the flask fitted with a calcium chloride drying tube. The reaction mixture was stirred overnight during which time a homogeneous solution formed. A solution of triethylamine (1.073g, 10.6mmol, 2.2 equiv.) in dry methanol (5mL) was added dropwise to the acid chloride over 30 minutes. The reaction mixture was stirred for a further two hours then the concentrated under vacuum. The residue was taken up in ethyl acetate (25mL) and washed with 1 M hydrochloric acid (3 x 20mL) and saturated sodium hydrogen carbonate solution (3 x 20mL). The organic layer was dried over magnesium sulphate then concentrated under vacuum to an off white crystalline solid 24, 1.019g (95%).
¹H NMR (*d₆*-DMSO) δ 7.63 (1 H, d, *J*= 4.0Hz, H-3), 7.35 (1H, d, *J*= 4.0Hz, H-4), 3.81 (3H, s, OCH₃); ¹³C NMR (*d₆*-DMSO) δ 160.7, 134.4 (CH), 134.0, 132.0 (CH), 119.6, 52.4 (CH₃); LCMS R_{T} = 3.57 min, (M⁺-Br) = 141.

### (b) Ethyl 5-(4-aminophenyl)thiophene-2-carboxylate (25)

Methyl 5-bromothiophene-2-carboxylate (0.916g, 4.1mmol) and 4-(4,4,5,5-tetramethyl-[1,3,2]dioxaboran-2-yl)-phenylamine (0.908g, 4.1 mmol, 1.0equiv.) were dissolved in a mixture of toluene (5mL), ethanol (5mL) and water (1 mL) and potassium carbonate (2.0g, 14.2mmol, 3.5equiv.) was added. The flask was purged with nitrogen gas then palladium tetrakis(triphenylphosphine) (0.1g) was added and the mixture heated at reflux for 72 hours. The reaction mixture was cooled to room temperature and diluted with ethyl acetate (50mL), the organic layer was separated and washed with water (2 x 50mL) then brine (50mL). The organic layerwas dried over magnesium sulphate and concentrated under vacuum. The residue was purified by column chromatography (silica gel, eluted with CHCl₃:MeOH 99.5:0.5). This gave the transesterified ethyl ester as a yellow solid 0.467g (46%).
¹H NMR (*d₆*-DMSO) δ 7.68 (1 H, d, J = 4.0Hz, H-3), 7.42 (2H, d, J = 8.6Hz, H-2',6'), 7.28 (1 H, d, J = 3.9Hz, H-4), 6.60 (2H, d, J = 8.6Hz, H-3'5'), 5.55 (2H, s, NH₂), 4.27 (2H, q, J = 7.1 Hz, OCH₂), 1.29 (3H, t, J = 7.1 Hz, CH₃); ¹³C NMR (*d₆*-DMSO) δ 161.5, 152.5, 150.0, 134.8 (CH), 128.2, 127.0 (CH), 121.1 (CH), 120.0, 113.9 (CH), 60.6 (CH₂), 14.2 (CH₃); LCMS R_{T} = 3.33 min, (M⁺+1) = 248.

### Example 10 - 4-(4-tert-Butoxycarbonylamino-phenyl)-1-methylpyrrole-2-caboxylic acid (32)

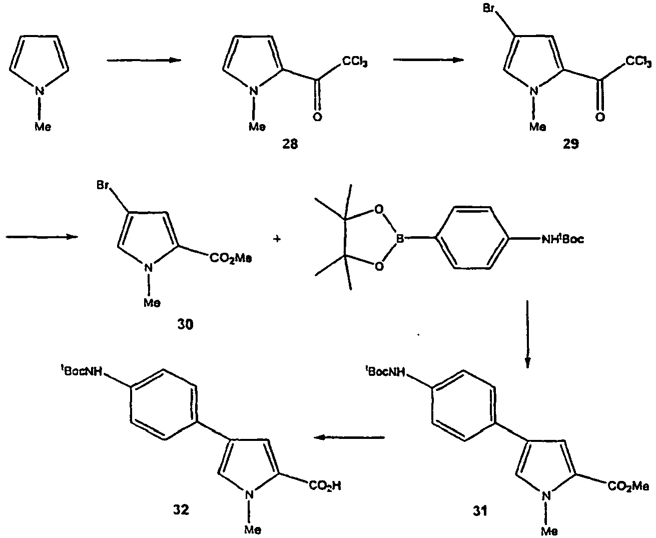

### (a) 1-Methyl-2-trichloroacetylpyrrole (28)

To a stirred solution of trichloroacetyl chloride (122.8 mL, 1.1 mol, 1 Equiv.) in dry Et₂O (300 mL) was added dropwise a solution of 1-methylpyrrole (98.7 mL, 1.11 mol, 1.01 Equiv.) in dry Et₂O (300 mL) over a period of 3 hours. Once the addition of the 1-methylpyrrole was complete it was stirred for a further 3 hours at room temperature. The reaction was quenched with the dropwise addition of potassium carbonate solution (80 g in 250 mL) (Caution: gas formation). The reaction mixture was then transferred to a separating funnel and the organic layer was separated and the aqueous layer extracted (3 x EtOAc). The combined organic layers were dried over MgSO₄ and concentrated under vacuum. The mixture was redissolved in ether and left to stand over night. The product crystallised as off white needles. (180 g, 72%) IR (film, cm⁻¹) 3299, 3121, 3008, 2954, 1789 (C=O), 1674, 1521, 1406, 1244, 1206, 1100, 980, 881, 757; ¹H NMR (CDCl₃) δ 7.42 (1 H, d, H-3), 6.89 (1 H, t, H-4), 6.15 (1 H, d, H-5), 3.90 (3H, s, CH₃); ¹³C NMR (CDCl₃) δ 133.6 (C4), 124.0 (C3), 122.4 (C2), 108.9 (C5), 38.5 (CH₃).

### (b) 4-Bromo-1-methyl-2-trichloroacetylpyrrole (29)

NBS (N-Bromosuccinimide, 2.36 g, 13.24 mmol, 1.0 Equiv.) was added to a stirred solution of 2-(trichloroacetyl)-1-methylpyrrole (3 g, 13.24 mmol, 1.0 Equiv.) in anhydrous THF (35 mL) at -10°C. The reaction mixture was kept at -10°C for 2 hours and then left to reach room temperature (ca. 4 h). The THF excess was evaporated in vacuum and the solid was redissolved in a mixture of EtOAc/Hexane (1:9). The resulting mixture was filtered through a plug of silica, and the filtrate was evaporated in vacuum. The resulting solid was recrystallised from hexane to give the product 29. (3.55 g, 88%) IR (film, cm⁻¹) 3148, 2956, 1669 (C=O), 1458, 1411, 1345, 1215, 1189, 1062, 992, 923, 842, 823, 785, 748, 714, 678. ¹H NMR (CDCl₃) rotamers δ 7.46 (78%) and 6.38 (22%) (1 H, d, *J* = 1.7 (78%) and 1.7 (22%)Hz, H-3), 6.95 (78%) and 5.88 (22%) (1 H, d, *J*= 1.5 (78%) and 1.-6 (22%) Hz, H-5), 3.95 (78%) and 2.88 (22%) (3H, s, NCH₃); ¹³C NMR (CDCl₃) rotamers δ 172.4 (C=O), 132.8 (C5), 124.6 (C3), 132.2 (C2), 96.1 (78%) and 95.7 (22%) (C4), 38.7 (CH₃); Elem. Anal. Calculated for C₇H₅BrCl₃O: C, 27.53; H, 1.65; N, 4.59. Found: C, 27.73; H, 1.62; N, 4.54.

### (c) Methyl 4-bromo-1-methylpyrrole-2-carboxylate (30)

To a stirred solution of 1-(4-Bromo-1-methyl-1*H*-pyrrol-2-yl)-2,2,2-trichloro-ethanone (3.28 g, 10.74 mmol, 1 Equiv.) in dry MeOH (30 mL) was added through a syringe a solution of sodium methoxide (0.5 mL). The sodium methoxide solution was prepared from NaH 60% in mineral oil (43 mg, 1.07 mmol, 0.1 Equiv.), which was previously washed with hexane. The solution was heated to reflux over a period of 30 minutes, when the TLC analysis showed complete consumption of the starting material. Few drops of concentrated H₂SO₄ were added to the solution to neutralise the base (pH 2). The excess MeOH was evaporated in vacuum and the resulting oil was redissolved in EtOAc (50 mL) and washed with water (40 mL). The aqueous layer was extracted with EtOAc (3 x 40 mL), and the organic phases were combined, dried (MgSO₄), filtered and concentrated in vacuum to afford the product 30 as a pale solid. (2.28 g, 97%) IR (film, cm⁻¹) 3138, 2948, 1692 (C=O), 1472, 1435, 1389, 1334, 1245, 1197, 1115, 1082, 1062, 921, 823, 807, 753; ¹H NMR (CDCl₃) δ 6.89 (1 H, d, J = 1.97Hz, H-3), 6.76 (1H, d, *J*= 1.93Hz, H-5), 3.89 (3H, s, NCH₃), 3.81 (3H, s, OCH₃); ¹³C NMR (CDCl₃) δ 160.8 (C=O), 128.7 (C5), 122.9 (C2), 119.2 (C3), 95.1 (C4), 51.2 (OCH₃) 36.9 (CH₃).

### (d) Methyl 4-(4-tert-Butoxycarbonylamino-phenyl)-1-methyl-pyrrole-2-caboxylate (31)

To a solution of 4-Bromo-1-methyl-1*H*-pyrrole-2-carboxylic acid methyl ester **30** (726 mg, 3.33 mmol, 1 Equiv.) in ethanol (6 mL) and toluene (4 mL) in a Emrys^{™} Process vial was added a solution of CsF (873 mg, 5.75 mmol, 1.0 Equiv.) in water (1.5 mL), *tert*-Butyl N-[4-(4,4,5,5-tetramethyl-1,3,2-dioxoborolan-2-yl)phenyl]carbamate (1.22 g, 3.83 mmol, 1.15 Equiv.) and Pd(PPh₃)₄ (64 mg, 0.07 mmol, 0.02 Equiv.) under nitrogen atmosphere and magnetic stirring. The vial was sealed with with a Reseal^{™} septa, and then the suspension was kept at 110°C for 30 minutes under microwave radiation¹ when the TLC showed no presence of starting material. Afterwards, water (50 mL) was added to the reaction, and it was extracted with EtOAc (3 x 40 mL), the filtrates were combined and dried over MgSO₄, and then concentrated under vacuum. The resulting oil was subject of flash chromatography (Hexane/EtOAc 9:1) to give the product 31 (440 mg, 40%). IR (film, cm⁻¹) 3353 (NH), 2975, 1696 (C=O), 1521, 1441, 1366, 1314, 1264, 1235, 1209, 1155, 1105, 1058, 822, 799, 657; ¹H NMR (CDCl₃) δ 7.40 (2H, d, *J*= 8.6Hz, H-2',6'), 7.33 (2H, d, *J* = 8.6Hz, H-3',5'), 7.16 (1 H, d, J = 2.0Hz, H-3), 7.02 (1 H, d, *J* = 2.0Hz, H-5), 6.45 (1 H, bs, NH), 3.95 (3H, s, NCH₃), 3.83 (3H, s, OCH₃), 1.52 (9H, s, [CH₃]₃); ¹³C NMR (CDCl₃) δ 161.7 (C=O), 152.8 (OC=ONH), 136.5 (C1'), 129.5 (C4'), 125.9 (C5), 125.6 (C2' and C6'), 123.7 (C4), 123.0 (C2), 119.0 (C5' and C3'), 114.6 (C3), 80.5 (OCquat), 51.1 (OCH₃) 36.9 (CH₃), 28.4 [CH₃]₃); MS (EI) *m*/*z* (relative intensity) 275.1 ([M - (CH₃)C]⁺ 100%), 331.2 ([M + H]⁺ 55%). ¹Emrys^{™} Optimizer microwave station (Personal Chemistry).

### (e) 4-(4-tert-Butoxycarbonylamino-phenyl)-1-methyl-pyrrole-2-caboxylic acid (32)

To a solution of 4-Bromo-1-methyl-1*H*-pyrrole-2-carboxylic acid methyl ester (726 mg, 3.33 mmol, 1 Equiv.) in ethanol (6 mL) and toluene (4 mL) in a Emrys^{™} Process vial was added a solution of CsF (873 mg, 5.75 mmol, 1.0 Equiv.) in water (1.5 mL), *tert*-Butyl N-[4-(4,4,5,5-tetramethyl-1,3,2-dioxoborolan-2-yl)phenyl]carbamate (1.22 g, 3.83 mmol, 1.15 Equiv.) and Pd(PPh₃)₄ (64 mg, 0.07 mmol, 0.02 Equiv.) under nitrogen atmosphere and magnetic stirring. The vial was sealed with with a Reseal^{™} septa, and then the suspension was kept at 110°C for 30 minutes under microwave radiation¹ when the TLC showed no presence of starting material. Afterwards, water (50 mL) was added to the reaction, and it was extracted with EtOAc (3 x 40 mL), the filtrates were combined and dried over MgSO₄, and then concentrated under vacuum. The resulting oil was subject of flash chromatography (Hexane/EtOAc 9:1) to give the product 32 (440 mg, 40%). IR (film, cm⁻¹): 3353 (NH), 2975, 1696 (C=O), 1521, 1441, 1366, 1314, 1264, 1235, 1209, 1155, 1105, 1058, 822, 799, 657. ¹H NMR (CDCl₃, 400 MHz) δ 1.52 (s, 9 H, CCH₃), 3.83 (s, 3 H, OCH₃), 3.95 (s, 3 H, CH₃), 6.45 (br s, 1 H, NH), 7.02 (d, 1 H, *J*= 2.03 Hz, H5), 7.16 (d, 1 H, *J* = 2.05 Hz, H3), 7.33 (d, 2 H, *J* = 8.58 Hz, H5' and H3'), 7.40 (d, 2 H, *J* 8.64 Hz, H2' and H6'). ¹³C NMR (CDCl₃, 400 MHz): δ 161.7 (C=O), 152.8 (OC=ONH), 136.5 (C1'), 129.5 (C4'), 125.9 (C5), 125.6 (C2' and C6'), 123.7 (C4), 123.0 (C2), 119.0 (C5' and C3'), 114.6 (C3), 80.5 (OCquat), 51.1 (OCH₃) 36.9 (CH₃), 28.4 (CCH₃). MS (EI) *m*/*z* (relative intensity) 275.1 ([M - (CH₃)C]⁺ 100%), 331.2 ([M + H]⁺ 55%).
¹Emrys^{™} Optimizer microwave station (Personal Chemistry).

### Example 11 - Methyl 4-(4-aminophenyl)-1-methyl-pyrrole-2-carboxylate (33)

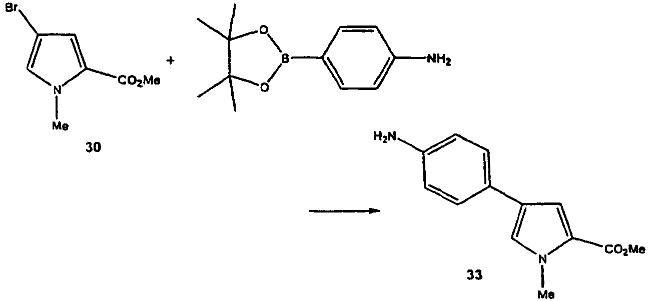

### (a) Methyl 4-(4-aminophenyl)-1-methyl-pyrrole-2-carboxylate (33)

To a solution of 4-Bromo-1-methyl-1*H*-pyrrole-2-carboxylic acid methyl **ester30** (7.46 g, 34.23 mmol, 1.5 Equiv.) in ethanol (20 mL) and toluene (12 mL) divided between two Emrys^{™} Process vial was added a solution of CsF (5.20 g, 34.23 mmol, 1.5 Equiv.) in water (4.0 mL) 4-(4,4,5,5-tetramethyl-1,3,2-dioxoborolan-2-yl)aniline (5 g, 22.82 mmol, 1.0 Equiv.) and Pd(PPh₃)₄ (791 mg, 0.68 mmol, 0.03 Equiv.) under nitrogen atmosphere and magnetic stirring. The vial was sealed with with a Reseal^{™} septa, and then the suspension was kept at 110°C for 20 minutes following further 20 minutes at 130°C under microwave radiation¹ when the TLC showed no presence of starting material. Afterwards, the TFA was added to the solution which was extracted with water (3x 50 mL). The aqueous phase was basified to pH 14 with NaOH, and it was extract with EtOAc (3x 60 mL), the filtrates were combined and dried over MgSO₄, and then concentrated under vacuum. The resulting oil was subject of flash chromatography (Hexane/EtOAc 9:1) to give the product 33 (440 mg, 8%). IR (film, cm⁻¹) 3366 (NH), 3374 (NH), 2987, 2945, 1688 (C=O), 1629, 1566, 1513, 1441, 1422, 1398, 1372, 1262, 1206, 1181, 1103, 1067, 951, 821, 784, 756; ¹H NMR (CDCl₃) δ 7.18 (2H, d, *J* = 8.5Hz, H2',6'), 7.11 (1H, d, *J* = 2.0Hz, H-3), 6.94 (1 H, d, *J* = 2.OHz, H-5), 6.66 (2H, d, *J* = 8.5Hz, H-3',5'), 3.92 (3H, s, CH₃), 3.82 (3H, s, OCH₃), 2.03 (2H, bs, NH); ¹³C NMR (CDCl₃) δ 161.7 (C=O), 144.8 (C1'), 126.2 (C2' and C6'), 125.5 (C5), 125.2 (C4'), 124.3 (C4), 122.7 (C2), 115.5 (C3' and C5'), 114.4 (C3), 51.1 (OCH₃) 36.8 (CH₃); MS (EI) *m*/*z* (relative intensity) 231.1 ([M + H]⁺ 100%).
¹Emrys^{™} Optimizer microwave station (Personal Chemistry).

### Example 12 - Methyl 4-(4-aminophenyl)-1-methylimidazole-2-carboxylate (35)

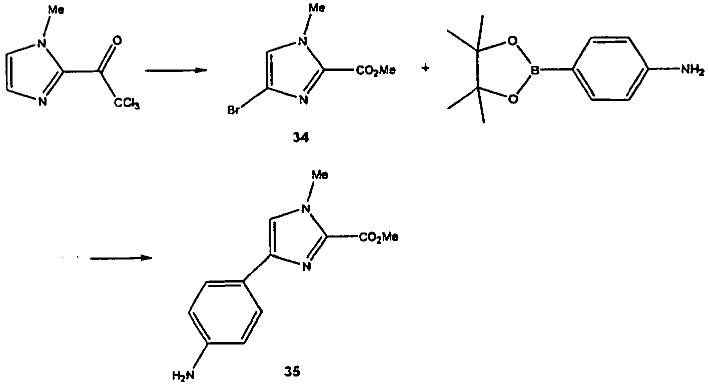

### (a) Methyl 4-bromo-1-methylimidazole-2-carboxylate (34)

NBS (N-Bromosuccinimide, 16.2 g, 91.0 mmol, 2.0 Equiv.) was added to a stirred solution of 2-(trichloroacetyl)-1-methylimidazole (as described by Nishiwaki, E.; Tanaka, S.; Lee, H. and Shibuya, M. Heterocycles, Vol. 27, No. 8, 1988, 1945-1952) (10.35 g, 45.5 mmol, 1.0 Equiv.) in anhydrous THF (180 mL) at-10°C. The reaction mixture was kept at -10°C for 2 hours and then left to reach room temperature (ca. 12 h). The THF excess was evaporated in vacuum and the solid was redissolved in chloroform and passed through a pad of Silica Gel (CHCl₃). The isolated compound was dried and redissolved in dried MeOH (100 mL). To the solution was added through a syringe a solution of sodium methoxide (5 mL). The sodium methoxide solution was prepared from NaH 60% in mineral oil (165 mg, 4.10 mmol, 0.1 Equiv.), which was previously washed with hexane. The solution was heated to reflux over a period of 30 minutes, when the TLC analysis showed complete consumption ofthe starting material. The excess of MeOH was evaporated in vacuum and the resulting oil was subject of a flash chromatography (CHCl₃/Hexane 8:2) to give a yellow solid **34.** (3.62 g, 36.3%). IR (film, cm⁻¹) 3121, 2949, 1718 (C=O), 1442, 1414, 1394, 1274, 1243, 1190, 1147, 1125, 1063, 950, 827, 804, 662, 631; ¹H NMR (CDCl₃) δ 7.05 (1H, s, H-5), 4.01 (3H, s, CH₃), 3.94 (3H, s, OCH₃); ¹³C NMR (CDCl₃) δ 158.6 (C=O), 136.0 (C2), 125.8 (C5), 115.6 (C4), 52.5 (OCH₃) 36.1 (CH₃); MS (EI) *m*/*z* (relative intensity) 219.01 ([M + H]⁺ 50%, 220.99 ([M + H]⁺ 50%).

### (b) Methyl 4-(4-aminophenyl)-1-methylimidazole-2-carboxylate (35)

To a solution of 4-Bromo-1-methyl-1*H*-imidazole-2-carboxylic acid methyl ester **34** (3.90 mg, 1.78 mmol, 1.0 Equiv.) in DMF (3 mL) in an Emrys^{™} Process vial was added CsF (405 mg, 2.67 mmol, 2.0 Equiv.) and 4-(4,4,5,5-tetramethyl-1,3,2-dioxoborolan-2-yl)aniline (429 mg, 1.96 mmol, 1.1 Equiv.) and Pd(PPh₃)₄ (61.7 mg, 0.053 mmol, 0.03 equiv.) under nitrogen atmosphere and magnetic stirring. The vial was sealed with Reseal^{™} septa, and then the suspension was kept at 110°C for 40 minutes. Afterwards, the DMF removed under vacuum and the resulting oil was subject of flash chromatography (CHCl₃/Hexane 8:2) to give 4-(4-amino-phenyl)-1-methyl-1*H*-imidazole-2-carboxylic acid methyl ester 35 as red-brown solid (67 mg, 16%). IR (film, cm⁻¹) 3468 (NH), 3325 (NH), 3202 (NH), 2955, 1697 (C=O), 1624, 1449, 1281, 1201, 1183, 1122, 1066, 950, 837, 783, 641; ¹H NMR (CDCl₃) δ 7.59 (2H, d, *J* = 8.5Hz, H2',6'), 7.19 (1H, s, H-5), 6.69 (2H, d, *J*= 8.5Hz, H-3',5'), 4.03 (3H, s, NCH₃), 3.96 (3H, s, OCH₃), 3.71 (2H, bs, NH); ¹³C NMR (CDCl₃): δ 159.7 (C=O), 146.0 (C1'), 142.4 (C4), 135.8 (C2), 126.5 (C2' and C6'), 123.6 (C4'), 121.0 (C5), 115.1 (C3' and C5'), 52.3 (OCH₃) 36.0 (CH₃); MS (EI) *m*/*z* (relative intensity) 232.03 ([M + H]⁺ 100%).

### Examples 13 - 4-(4-tert-Butoxycarbonylamino-phenyl)-1-methylimidazole-2-carboxylic acid (37)

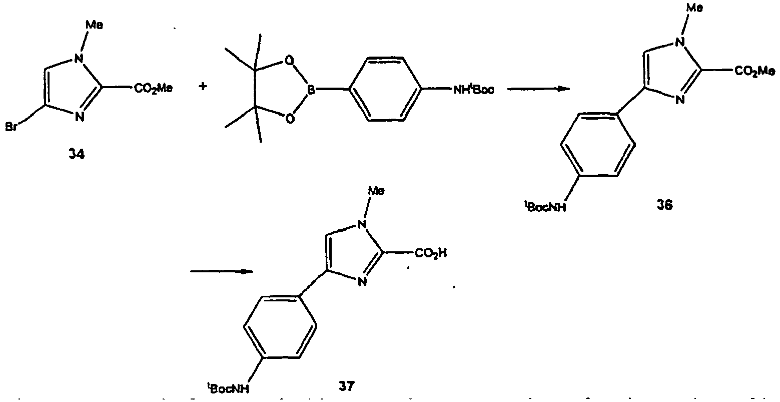

### (a) Methyl 9-(4-tert-butoxycarbonylamino-phenyl)-1-methylimidazole-2-carboxylate (36)

To a solution of 4-Bromo-1-methyl-1*H*-imidazole-2-carboxylic acid methyl ester **34** (352.8 mg, 1.61 mmol, 1.0 Equiv.) in DMF -(6 mL) in an Emrys^{™} Process vial was added CsF (489 mg, 3.22 mmol, 2.0 Equiv.) and *tert*-Butyl N-[4-(4,4,5,5-tetramethyl-1,3,2-dioxoborolan-2-yl)phenyl]carbamate (617 mg, 1.93 mmol, 1.2 Equiv.) and Pd(PPh₃)₄ (56 mg, 0.048 mmol, 0.03 Equiv.) under nitrogen atmosphere and magnetic stirring. The vial was sealed with Reseal^{™} septa, and then the suspension was kept at 110°C for 40 minutes. Afterwards, the DMF removed under vacuum and the resulting oil was subject of flash chromatography (Hexane/EtOAc 8:2) to give 4-(4-*tert*-butoxycarbonylamino-phenyl)-1-Methyl-1*H*-imidazole-2-carboxylic acid methyl ester **36** as a yellow glass (85 mg, 16%). IR (film, cm⁻¹) 3355 (NH), 2982, 1692 (C=O), 1516, 1461, 1400, 1365, 1319, 1285, 1267, 1234, 1204, 1153, 1125, 1056, 949, 905, 834, 790, 652, 632; ¹H NMR (CDCl₃) δ 7.72 (2H, d, *J*= 8.6Hz, H2',6'), 7.38 (2H, d, J= 8.5Hz, H3',5'), 7.27 (1H, s, H-5), 6.56 (1H, bs, NH), 4.04 (3H, s, NCH₃), 3.97 (3H, s, OCH₃), 1.52 (9H, s, [CH₃]₃); ¹³C NMR (CDCl₃): δ 159.7 (C=O), 152.6 (OC=ONH), 141.7 (C4), 137.8 (C1'), 136.1 (C2), 127.8 (C4'), 125.9 (C2' and C6'), 121.8 (C5), 118.4 (C3' and C5'), 76.7 ([CH₃]₃), 52.4 (OCH₃) 36.0 (CH₃), 28.3 ((CH₃)₃); MS (EI) *m*/*z* (relative intensity) 332.1 ([M + H]⁺ 100%).

### (b) 4-(4-tert-Butoxycarbonylamino-phenyl)-1-methyl-imidazole-2-carboxylic acid (37)

To a suspension of 4-(4-tert-Butoxycarbonylamino-phenyl)-1-methyl-1*H*-imidazole-2-carboxylic acid methyl ester **36** (50 mg, 0.15 mmol, 1.0 Equiv.) in methanol (0.2 mL), in a Emrys^{™} Process vial (0.2 - 0.5 mL) with magnetic bar, was added 0.5 mL of an aqueous solution of KOH (8.5 mg, 0.15 mmol, 1.0 Equiv.). The vial was sealed with Reseal^{™} septa, and then the suspension was kept at 100°C for 7 minutes under microwave radiation¹ when the TLC showed no presence of starting material. Afterwards, the solvents were removed under vacuum and water (1.0 mL) was added to the reaction which acidified to pH 7 with HCl 50%. The resulting solid was subject of flash chromatography (EtOAc/ MeOH 9:1) to give a white solid 37 (38 mg, 90%). IR (disc, cm⁻¹) 3385 (br OH), 2978, 1712 (C=O), 1599 (C=O), 1525, 1455, 1345, 1315, 1238, 1157, 1050, 1024, 963, 824, 808, 766, 642; ¹H NMR (CDCl₃) δ 9.37 (1H, bs, NH), 8.41 (1H, s, COOH), 7.61 (2H, d, J= 8.4Hz, H2',6'), 7.48 (1H, s, H-5), 7.41 (2H, d, *J* = 8.4Hz, H3',5'), 3.94 (3H, s, NCH₃), 1.47 (9H, s, [CH₃]₃); ¹³C NMR (CDCl₃) δ 162.2 (C=O), 152.7 (OC=ONH), 144.9 (C2), 138.0 (C1'), 137.8 (C4), 127.5 (C4'), 125.3 (C2' and C6'), 121.0 (C5), 117.8 (C3' and C5'), 78.9 (C(CH₃)₃), 35.2 (CH₃), 28.1 ([CH₃]₃); MS (EI) *m*/*z* (relative intensity) 318.07 ([M + H]⁺ 100%).
¹Emrys^{™} Optimizer microwave station (Personal Chemistry).

### Example 14 - General method for the synthesis of amides of 5-(4-nitrophenyl)-furan-2-carboxylic acid

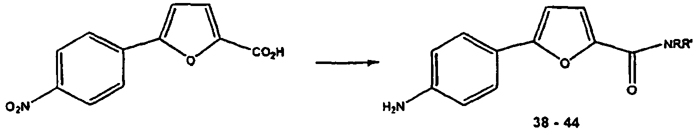

Oxalyl chloride (1.70 mL, 19.47 mmol, 1.3 Equiv) and DMF (catalytic) were added to a solution/suspension of 4-nitrophenylfurane-2-carboxylic acid in 200 mL of DCM. The mixture was stirred at room temperature for 20 hours. DCM and oxalyl chloride excess were evaporated off in vacuum, the resulting yellow solid was split equally in seven two-necked round flask. The chloride was redissolved in THF (10 mL) and the respective amine solutions (see table below) in THF (10 mL) were added dropwise. TEA (1.0 Equiv.) was also added to the following entries: 3-dimethylaminopropyl amine, morpholine and 3-morpholino propylamine. The mixtures were allowed to stirr at room temperature for 4 hours, and then the excess solvents were evaporated under vacuum. The crude materials (low impurity rates, see HPLCs) were redissolved in EtOH (10-20 mL) and they were transferred to appropriate flasks to undergo catalytic hydrogenation under H₂ in a Parr apparatus (10% Pd/C, 20psi average) for various times. The mixture was filtered over celite and the solvent removed at reduced pressure to afford the respective amides.

Amines used in the above procedure:

| Amine | Stoichiometry |
|---|---|
| Ammonia | 0.5 M in dioxane (25.7 mL, 12.84 mmols, 6.0 Equiv.) |
| Methylamine | 2.0 M in THF (6.42 mL, 12.84 mmols, 6.0 Equiv.) |
| Diethylamine | 2.0 M in THF (6.42 mL, 12.84 mmols, 6.0 Equiv.) |
| Morpholine | 0.56 mL, 6.42 mmols, 3.0 Equiv. |
| 1,4-diaminobutane | 0.11 mL, 1.07 mmols, 0.5 Equiv. |
| 3-dimethylaminopropylamine | 0.35 mL, 2.78 mmols, 1.3 Equiv. |
| 3-morpholinepropylamine | 0.94 mL, 6.42 mmols, 3.0 Equiv. |

Compounds prepared using this method:

| Compound | Amide substituent |
|---|---|
| 38 | -NH₂ |
| 39 | -NHMe |
| 40 | -NMe₂ |
| 41 | 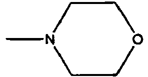 |
| 42 | 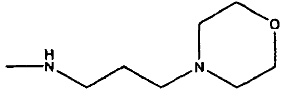 |
| 43 | 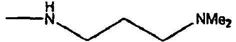 |

Characterising data for the compounds:

### (a) 5-(4-Aminophenyl)-furan-2-carboxylic acid amide (38)

Purified by flash chromatography. Yield 86 mg, 20%. ¹H NMR (*d₆*-Acetone) δ 7.60 (2H, dt, *J*= 2.4, 8.6Hz, H2', 6'), 7.20 - 7.40 (1 H, bs, NH amide rotamer), 7.08 (1H, d, *J*= 3.5Hz, H-3), 6.73 (2H, dt, *J*= 2.4, 8.6Hz, H3',5'), 6.65 (1 H, d, *J*= 3.5Hz, H-4), 6.40 - 6.60 (1 H, bs, NH amide rotamer), 4.97 (2H, bs, NH₂) ; ¹³C NMR (*d₆*-Acetone) δ 160.4 (C=ONH), 157.5 (C5), 150.2 (C2), 147.1 (C4'), 126.6 (C2' and C6'), 120.7 (C1'), 116.8 (C3), 115.0 (C3' and C5'), 104.5 (C4); MS (EI) *m*/*z* (relative intensity) 203.00 ([M + H]⁺ 100%).

### (b) 5-(4-Aminophenyl)-furan-2-carboxylic acid methylamide (39)

Purified by crystallization. Yield 273 mg, 59%. MS (EI) m/z (relative intensity) 217.02 ([M + H]⁺ 100%).

### (c) 5-(4-Aminophenyl)-furan-2-carboxylic acid dimethylamide (40)

Purified by crystallization. Yield 252 mg, 51%. MS (EI) *m*/*z* (relative intensity) 231.04 ([M + H]⁺ 100%).

### (d) Morpholin-yl-[5-(4-aminophenyl)-furan-2-yl]-methanone (41)

Purified by crystallization. Yield 332 mg, 57%. MS (EI) m/z (relative intensity) 272.97 ([M + H]⁺ 100%).

### (e) 5-(4-Aminophenyl)-furan-2-carboxylic acid (4-morpholin-4-yl-propyl)-amide (42)

Purified by flash chromatography. Yield 532 mg, 75%. ¹H NMR (*d₆*-Acetone) δ 7.84 (1H, bs, NH), 7.57 (2H, dt, *J*= 2.5, 8.7Hz, H2',6'), 7.04 (1 H, d, *J*= 3.5Hz, H-3), 6.73 (2H, dt, *J*= 2.5, 8.7Hz, H3',5'), 6.65 (1 H, d, *J*= 3.5Hz, H-4), 5.02 (2H, bs, NH₂), 3.63 (4H, t, *J* = 4.6Hz, O(CH₂)₂), 3.45 (2H, q, *J* = 5.9Hz, CH₂CH₂NHCO), 2.40 - 2.45 (6H, m, morpholine-CH₂CH₂ and (CH₂)₂N), 1.78 (2H, quintuplet, *J*= 6.8Hz, CH₂CH₂CH₂); MS (EI) *m*/*z* (relative intensity) 329.98 ([M + H]⁺ 100%).

### (f) 5-(4-Aminophenyl)-furan-2-carboxylic acid (3-dimethylaminopropyl)amide (43)

Purified by flash chromatography. Yield 446 mg (91% purity), 71%. MS (EI) *m*/*z* (relative intensity) 288.04 ([M + H]⁺ 100%).

### (g) Buty1-N,N-1,4-bis[5-(4-Aminopheny/)-furan-2-carboxamide] (44)

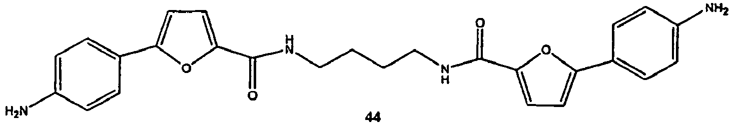

Purified by recrystallization. Yield 329 mg of 44, 33.5%. MS (EI) m/z (relative intensity) 459.05 ([M + H]⁺ 100%).

### Example 15 - Ethyl 2-{4-[(4-tert-butoxycarbonylamino-1-methyl-1H-pyrrole-2-carbonyl)-amino]-phenyl}-thiazole-4-carboxylate (27)

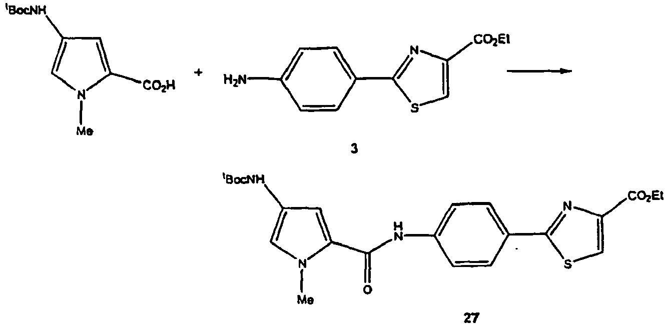

A solution/suspension of the biaryl amino ester (0.103g, 0.42mmol) and the Boc pyrrole acid (0.100g, 0.42mmol, 1 equiv.) in dry dichloromethane (5mL) was treated with EDCI (0.159g, 0.83mmol, 2 equiv.) then DMAP (0.127g, 1.04mmol, 2.5 equiv.). The reaction mixture was stirred at room temperature for 72 hours. During this time a precipitate formed which was collected on a filter and washed with dichloromethane (3 x 2mL) then dried under vacuum. This gave the product as a white solid, 0.105g, (53%). LCMS R_{T} = 3.78 min, (M⁺+1) = 471.

### Example 16 - General method for the synthesis of Boc protected biaryl-pyrrole-pyrrole conjugates

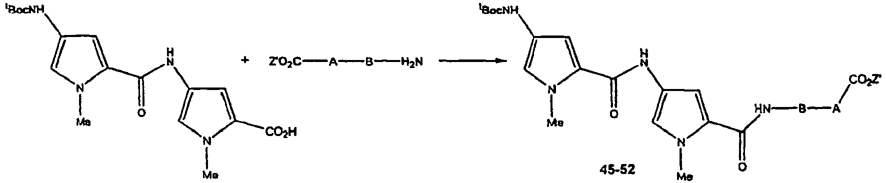

A solution of the biaryl amino ester (0.41 mmol) in dry dichloromethane (5mL) was treated with the dipyrrole acid (0.150g, 0.41 mmol, 1.0 equiv.), then EDCI (0.159g, 8.2mmol, 2.0 equiv.) and DMAP (0.126g, 1.0 mmol, 2.5 equiv.). The reaction mixture was stirred over night then the solvent was removed under vacuum. The residue was dissolved/suspended in ethyl acetate (20mL) and washed with either water (imidazole, pyridine containing biaryls) or 1 M HCI (others) (3 x 15mL), then saturated sodium hydrogen carbonate solution (3 x 15mL). The organic layer was dried over MgSO₄ and the solvent removed under vacuum. The resulting foam was then dried under vacuum.

Compounds prepared using the above method:

| Compound | -Biaryl-CO-Z' |
|---|---|
| 45 | 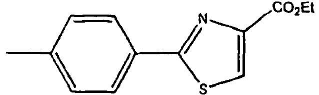 |
| 46 | 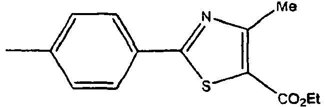 |
| 47 | 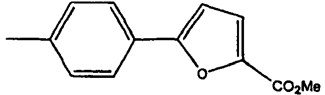 |
| 48 | 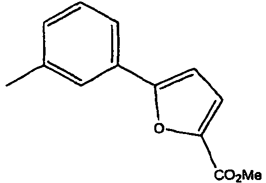 |
| 17 | 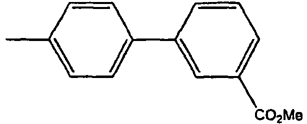 |
| 49 | 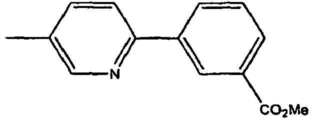 |
| 50 | 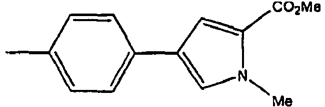 |
| 51 | 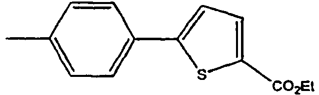 |
| 52 | 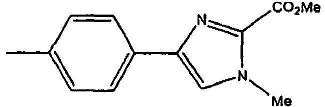 |

### Characterising data for the compounds prepared using this method:

### (a) Ethyl 2-[4-({4-[(4-tert-butoxycarbonylamino-1-methyl-1H-pyrrole-2-carbonyl)-amino]-1-methyl-1H-pirrole-2-carbonyl}-amino)-phenyl]-thiazole-4-carboxylate (45)

The yield was 0.193g (80%) of yellow foam. ¹H NMR (*d₆*-Acetone) δ 9.43 (1 H, bs, NH), 9.24 (1 H, bs, NH), 8.35 (1H, s, thiazole-H), 8.09 (1H, bs, NH), 7.99 (4H, m, phenyl-H), 7.30 (1H, d, *J*= 1.8Hz, pyrrole-H), 7.15 (1H, d, *J* = 1.8Hz, pyrrole-H), 6.93 (1H, s, pyrrole-H), 6.78 (1H, s, pyrrole-H), 3.96 (3H, s, NCH₃), 3.93 (3H, s, NCH₃), 4.38 (2H, q, *J* = 7.1Hz, CH₂), 1.47 (9H, s, [CH₃]₃), 1.38 (3H, t, *J* = 7.1Hz, CH₃); LCMS R_{T} = 3.72 min, (M⁺+1) = 593.

### (b) Ethyl 2-[4-({4-[(4-tert-butoxycarbonylamino-1-methyl-1H-pyrrole-2-carbonyl)-amino]-1-methyl-1H-pyrrrole-2-carbonyl}-amino)-phenyl]-4-methyl-thiazole-5-carboxylate (46)

The yield was 0.204g (90%) of yellow foam. ¹H NM R (*d₆*-Acetone) δ 9.45 (1H, bs, NH), 9.24 (1 H, bs, NH), 8.09 (1H, bs, NH), 7.98 (4H, m, phenyl-H), 7.30 (1H, d, *J*= 1.8Hz, pyrrole-H), 7.15 (1H, d, *J*= 1.8Hz, pyrrole-H), 6.93 (1H, s, pyrrole-H), 6.79 (1 H, s, pyrrole-H), 4.34 (2H, t, *J* = 7.1 Hz, OCH₂), 3.96 (3H, s, NCH₃), 3.93 (3H, s, NCH₃), 2.71 (3H, s, thiazole-CH₃), 1.46 (9H, s, [CH₃]₃), 1.36 (3H, t, J = 7.1Hz, CH₃); LCMS R_{T} = 4.00 min, (M⁺+1) = 607.

### (c) Methyl 5-[4-({4-[(4-tert-butoxycarbonylamino-1-methyl-1H-pyrrole-2-carbonyl)-amino]-1-methyl-1H-pyrrole-2-carbonyl}-amino)-phenyl]-furan-2-carboxylate (47)

The yield was 0,118g, (54%). ¹H NMR (*d₆*- Acetone) δ 9.35 (1 H, s, NH), 9.23 (1 H, s, NH), 8.09 (s, 1 H, bs, NH), 7.94 (2H, d, *J* = 8.7Hz, phenyl-H), 7.79 (2H, d, *J*= 8.7Hz, phenyl-H), 7.30 (1H, d, *J*= 3.6Hz, furan-H-3), 7.29 (1H, d, *J* = 1.7Hz, pyrrole-H), 7.13 (1H, d, *J*= 1.5Hz, pyrrole-H), 6.95 (1H, d, *J* = 3.6Hz, furan-H-4), 6.93 (1H, s, pyrrole-H), 6.78 (1H, s, pyrrole-H), 3.95 (s, 3H, s, NCH₃), 3.93 (3H, s, NCH₃), 3.87 (3H, s, OCH₃), 1.46 (9H, s, [CH₃]₃); LCMS R_{T} = 3.72 min, (M⁺+1) = 562.

### (d) Methyl 5-[3-({4-[(4-tert-butoxycarbonylamino-1-methyl-1H-pyrrole-2-carbonyl)-amino]-1-methyl-1H-pyrrole-2-carbonyl}-amino)-phenyl]-furan-2-carboxylate (48)

The yield was 0.200g (91%) of yellow foam. LCMS R_{T} = 3.72 min, (M⁺+1) = 562.

### (e) Methyl 4'-({4-[(4-tert-butoxycarbonylamino-1-methyl-1H-pyrrole-2-carbonyl)-amino]-1-methyl-1H-pyrrole-2-carbonyl}-amino)-biphenyl-3-carboxylate (17)

The yield was 0.156g (66%) of yellow foam. ¹H NMR (*d₆*- Acetone) δ 9.31 (1H, bs, NH), 9.23 (1H, bs, NH), 8.27 (1H, t, *J* = 1.7Hz, biphenyl-H-2), 8.09 (1 H, bs, NH), 7.98 (1 H, d, *J* = 1.2Hz, biphenyl-H-4/6), 7.95 (1 H, d, *J* = 8.7Hz, biphenyl-H-2'/6'), 7.92 (1H, d, *J*= 1.9Hz, biphenyl-H-4/6), 7.68 (1 H, d, *J* = 8.7Hz, biphenyl-H-3'/5'), 7.59 (1 H, t, *J*= 7.7Hz, biphenyl-H-5), 7.29 (1 H, d, *J* = 1.8Hz, pyrrole-H), 7.12 (1H, d, *J* = 1.8Hz, pyrrole-H), 6.93 (1 H, s, pyrrole-H), 6.79 (1 H, s, pyrrole-H), 3.96 (3H, s, NCH₃), 3.93 (3H, s, NCH₃), 3.93 (3H, s, OCH₃), 1.47 (9H, s, [CH₃]₃); LCMS R_{T} = 3.88 min, (M⁺+1) = 572.

### (f) Methyl 3-[5-({4-[(4-tert-butoxycarbonylamino-1-methyl-1H-pyrrole-2-carbonyl)-amino]-1-methyl-1H-pyrrole-2-carbonyl}-amino)-pyridin-2-yl]-benzoate (49)

The yield was 0.232g (98%) of yellow foam. ¹H NMR (*d₆*- Acetone) δ 9.51 (1 H, bs, NH), 9.26 (1 H, bs, NH), 9.04 (1 H, d, J = 2.0Hz, pyridinyl-H-6), 8.78 (1 H, t, J = 1.7Hz, phenyl-H-2), 8.44 (1 H, m, phenyl-H-4/6), 8.36 (1 H, m, phenyl-H-4/6), 8.12 (1H, bs, NH), 8.04 (1 H, m, pyridinyl-H-4), 7.99 (1 H, d, J = 8.6Hz, pyridinyl-H-3), 7.62 (1 H, t, J = 7.8Hz, phenyl-H-5), 7.30 (1 H, d, J = 1.8Hz, pyrrole-H), 7.19 (1 H, d, J = 1.8Hz, pyrrole-H), 6.94 (1 H, s, pyrrole-H), 6.79 (1H, s, pyrrole-H), 3.97 (3H, s, NCH₃), 3.94 (3H, s, NCH₃), 3.94 (3H, s, OCH₃), 1.47 (9H, s, [CH₃]₃); LCMS R_{T} = 3.60 min, (M⁺+1) = 573.

### (g) Methyl 4-[4-({4-[(4-tert-butoxycarbonylamino-1-methyl-1H-pyrrole-2-carbonyl)-amino]-1-methyl-1H-pyrrole-2-carbonyl}-amino)-phenyl]-1-methyl-1H-pyrrole-2-carboxylate (50)

LCMS R_{T} = 3.73 min, (M⁺+1) = 575. Used directly in the next step without further purification.

### (h) Ethyl 5-[4-({4-[(4-tert-butoxycarbonylamino-1-methyl-1H-pyrrole-2-carbonyl)-amino]-1-methyl-1H-pyrrole-2-carbonyl}-amino)-phenyl]-thiophene-2-carboxylate (51)

The product was purified by column chromatography. The yield was 0.102g (42%).
¹H NMR (*d₆*- Acetone) δ 9.37 (1H, bs, NH), 9.25 (1H, bs, NH), 8.11 (1H, bs, NH), 7.92 (2H, d, *J* = 8.7Hz, phenyl-H-2,6), 7.76 (1 H, d, *J* = 3.9Hz, thiophene-H-3), 7.71 (2H, d, *J*= 8.7Hz, phenyl-H-3,5), 7.46 (1 H, d, *J* = 3.9Hz, thiophene-H-4), 7.28 (1 H, d, *J* = 1.7Hz, pyrrole-H), 7.12 (1 H, d, *J*= 1.6Hz, pyrrole-H), 6.93 (1 H, s, pyrrole-H), 6.79 (1H, s, pyrrole-H), 4.34 (2H, q, *J* = 7.1 Hz, OCH₂), 3.95 (3H, s, NCH₃), 3.93 (3H, s, NCH₃), 1.46 (9H, s, [CH₃]₃), 1.36 (3H, t, *J* = 7.1Hz, CH₂CH₃); LCMS R_{T} = 3.98 min, (M⁺+1) = 592.

### (i) Methyl 4-[4-({4-[(4-tert-butoxycarbonylamino-1-methyl-1H-pyrrole-2-carbonyl)-aminol-1-methyl-1H-pyrrole-2-carbonyl}-amino)-phenyl]-1-methyl-1H-imidazole-2-carboxylate (52)

The reaction was performed on a 1.5mmol scale. The yield was 0.069g, (81%) of yellow foam. ¹H NMR *(d₆*-Acetone) δ 9.28 (1 H, bs, NH), 9.24 (1 H, bs, NH), 8.15 (1 H, bs, NH), 7.82 (4H, m, phenyl-H), 7.73 (1H, s, imidazole-H), 7.29 (1 H, d, J = 1.8Hz, pyrrole-H), 7.08 (1 H, d, J = 1.7Hz, pyrrole-H), 6.94 (1 H, s, pyrrole-H), 6.78 (1 H, s, pyrrole-H), 4.05 (3H, s, NCH₃), 3.95 (3H, NCH₃), 3.93 (3H, s, NCH₃), 3.88 (3H, s, OCH₃), 1.46 (9H, s, [CH₃]₃); LCMS R_{T} = 3.22 min, (M⁺+1) = 576.

### Example 17-Synthesis of Ethyl2-(4-{[4-(4-dimethylaminobutyrylamino)-methyl-1H-pyrrole-2-carbonyl]-aminol-phenyl)-thiazole-4-carboxylate (26)

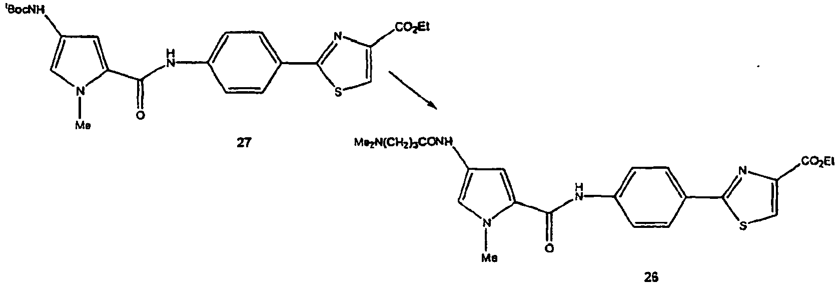

The Boc protected amine (0.05g, 0.11 mmol), was dissolved in dry THF (1 mL) and 4M HCl in dioxane (2mL) was added. The reaction mixture was stirred at room temperature for 2 hours then the solvent was removed under vacuum and the residue dried under vacuum. The resulting solid was dissolved in dry DMF (2mL) and 4-[N,N-dimethylamino]butyric acid (0.036g, 0.22mmol, 2 equiv.) was added followed by EDCI (0.041 g, 0.21 mmol, 2 equiv.) and DMAP (0.026g, 0.21 mmol, 2 equiv.). The reaction mixture was stirred overnight then the DIM was removed under a stream of nitrogen. The residue was dissolved in water and basified with 15% aqueous sodium hydroxide solution (-3 drops). The aqueous layer was extracted with ethyl acetate (4 x 10mL) and the combined organic layers were dried over magnesium sulphate then concentrated under vacuum. The residue was suspended in ether (10mL) and dispensed into 1.5mL Eppendorf tubes (x 10) and centrifuged for 90 seconds. The ether was removed and the solid resuspended in ether (10 x 1 mL) and the tubes centrifuged for a further 90 seconds. The ether was again removed and the solid dried under a stream of nitrogen, then under vacuum. This gave an off white solid, 0.030g (58%). ¹H NMR (*d₆*- Acetone) δ 9.41 (1 H, bs, NH), 9.33 (1 H, bs, NH), 8.35 (1 H, s, thiazole-H), 7.97 (4H, m, phenyl-H), 7.25 (1H, d, *J* = 1.8Hz, pyrrole-H), 6.99 (1 H, d, *J* = 1.8Hz, pyrrole-H), 4.37 (2H, q, *J* = 7.1 Hz, OCH₂), 3.94 (3H, s, NCH₃), 2.33 (4H, 2 x t, *J* = 7.0Hz, sidechain-H-2,4), 2.21 (6H, s, N[CH₃]₂), 1.81 (2H, p, *J* = 7.0Hz, sidechain-H-3), 1.38 (3H, t, *J* = 7.2Hz, CH₂CH₃); LCMS R_{T} = 0.58 min, (M⁺+1) = 484.

### Example 18 - General method for the synthesis of biaryl-pyrrole-pyrrole-charged tail conjugates

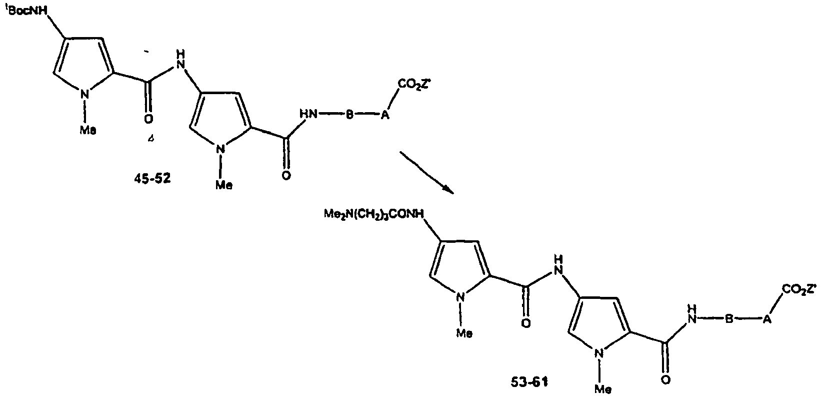

The Boc protected amine (0.050g) in a dry round bottomed flask dissolved in dry THF (0.5mL) and then treated with 4M HCl in dioxane (2mL). The reaction mixture was stirred for 1 hour during which time a precipitate formed. The solvent was removed under vacuum and the residue dried under vacuum. The resulting solid was then dissolved in dry DMF (1 mL) and *4*-*[N,N*-dimethylamino]butyric acid (2 equiv.) added followed by EDCI (2 equiv.) and DMAP (2.5 equiv.). The resulting solution was stirred under a nitrogen atmosphere overnight then the DMF was removed under a stream of nitrogen and the residue dried under vacuum. The residue was dissolved in water (10mL) and made slightly alkaline by the addition of 15% aqueous sodium hydroxide solution (3-5 drops). The aqueous phase was extracted with ethyl acetate (4 x 1 0mL) The organic layers combined, dried over MgSO₄ and concentrated under vacuum. The residue was suspended in ether (10mL) and dispensed into 1.5mL Eppendorf tubes (x 10) and centrifuged for 5 minutes. The ether was removed and the solid resuspended in ether (10 x 1 mL) and centrifuged for5 minutes. The ether was again removed and the solid dried under a stream of nitrogen gas then under vacuum to give the product.

The following compounds were prepared using the above method:

| Compound | -B-A-CO-Z' |
|---|---|
| 53 | 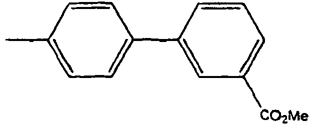 |
| 54 | 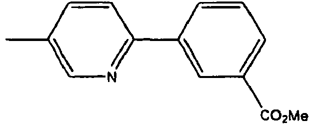 |
| 55 | 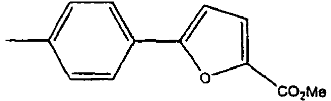 |
| 56 | 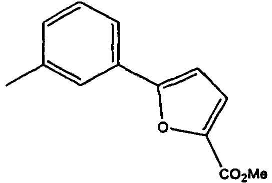 |
| 57 | 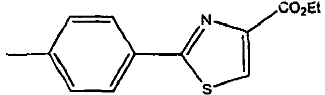 |
| 58 | 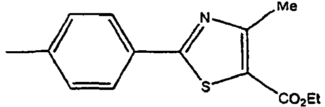 |
| 59 | 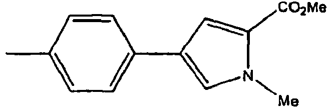 |
| 60 | 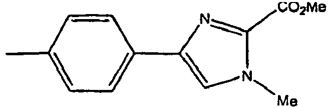 |
| 61 | 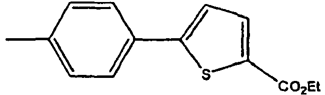 |

Characterising data for the compounds prepared using this method:

### (a) Methyl 4'-5[4-{[4-(4-Dimethylamino-butyrylamino)-1-methyl-1H-pyrrole-2-carbonyl]-amino}-1-methyl-1H-pyrrole-2-carbonyl)-amino]biphenyl-3-carboxylate (53)

The yield of white solid was 0.051 g (99%). ¹H NMR *(d₆-* Acetone) δ 9.36 (1 H, bs, NH), 9.29 (1 H, bs, NH), 9.24 (1 H, bs, NH), 8.27 (1 H, t, J =1.6Hz, biphenyl-H-2), 8.00-7.90 (2H, m, biphenyl-H-4/6 ), 7.96 (2H, d, J = 8.8Hz, biphenyl-H-2'/6'), 7.68 (2H, d, J = 8.7Hz, biphenyl-H-3'/5'), 7.60 (1 H, t, J = 7.8Hz, biphenyl-H-5), 7.29 (1 H, d, J = 1.7Hz, pyrrole-H), 7.18 (1H, d, J = 1.7Hz, pyrrole-H), 7.11 (1 H, d, J = 1.8Hz, pyrrole-H), 6.82 (1 H, d, J = 1.8Hz, pyrrole-H), 3.96 (3H, s, NCH₃), 3.93 (3H, s, NCH₃), 3.92 (3H, s, OCH₃), 2.31 (4H, 2xt, J = 7.0Hz, sidechain-H-2/4), 2.19 (6H, s, N[CH₃]₂), 1.78 (2H, p, J = 7.0Hz, sidechain-H-3); LCMS R_{T} = 2.50 min, (M⁺+1) = 585.

### (b) Methyl 3-{5-[(4-{[4-(4-Dimethylamino-butyrylamino)-1-methyl-1H-pyrrole-2-carbonyl]-amino}-1-methy/-1H-pyrro/e-2-carbonyl)-amino]-pyridine-2-yl}-benzoate (54)

The yield was 0.039g (77%) of off white solid. ¹H NMR (*d₆*-Acetone) δ 9.56 (1H, bs, NH), 9.30 (1H, bs, NH), 9.26 (1 H, bs, NH), 9.04 (1 H, d, J = 2.3Hz, pyridinyl-H-6), 8.78 (1 H, t, J = 1.6Hz, phenyl-H-2), 8.44 (1 H, m, phenyl-H-4/6), 8.35 (1 H, m, phenyl-H-4/6), 8.03 (1 H, m, pyridinyl-H-4), 7.99 (1 H, d, J = 8.7Hz, pyridinyl-H-3), 7.62 (1 H, t, J = 7.8Hz, phenyl-H-5), 7.30 (1 H, d, J = 1.7Hz, pyrrole-H), 7.18 (2H, m, pyrrole-H), 6.83 (1 H, d, J = 1.8Hz, pyrrole-H), 3.97 (3H, s, NCH₃), 3.94 (3H, s, NCH₃), 3.93 (3H, s, OCH₃), 2.31 (4H, 2xt, J = 7.0Hz, sidechain-H-2/4), 2.19 (6H, s, N[CH₃]₂), 1.77 (2H, p, J = 7.0Hz, sidechain-H-3); LCMS R_{T} = 2.13 min, (M⁺+1) = 586.

### (c) Methyl 5-{4-[(4-{[4-(4-Dimethylamino-butytylamino)-1-methyl-1H-pyrrole-2-carbonyl]-amino)-1-methyl- H-pyrrole-2-carbonyl)-aminol-phenyl)-furan-2-carboxylate (55)

The yield was 0.032g (61 %) of off white solid. ¹H NMR (d₆-Acetone) δ 9.41 (1 H, bs, NH), 9.26 (1H, bs, NH), 9.23 (1 H, bs, NH), 7.94 (2H, J = 8.8Hz, phenyl-H-2,6), 7.80 (2H, d, J = 8.8Hz, phenyl-H-3,5), 7.31 (1 H, d, J = 3.6Hz, furan-H-3), 7.29 (1 H, d, J = 1.7Hz, pyrrole-H), 7.17 (1 H, d, J = 1.7Hz, pyrrole-H), 7.11 (1 H, d, J = 1.8Hz, pyrrole-H), 6.97 (1 H, d, J = 3.6Hz, furan-H-4), 6.81 (1 H, d, J = 1.8Hz, pyrrole-H), 3.95 (3H, s, NCH₃), 3.93 (3H, s, NCH₃), 3.87 (3H, s, OCH₃), 2.30 (4H, 2xt, J = 7.1 Hz, sidechain-H-2,4), 2.18 (6H, s, N[CH₃]₂), 1.79 (2H, p, J = 7.0Hz, sidechain-H-3); LCMS R_{T} = 2.27 min, (M⁺+1) = 575.

### (d) Methyl 5-{3-[(4-{[4-(4-Dimethylamino-butyrylamino)-1-methyl-1H-pyrrole-2-carbonyl]-amino}-1-methyl-1H-pyrrole-2-carbonyl)-amino]-phenyl}-furan-2-carboxylate (56)

¹H NMR (*d₆*- Acetone) δ 9.43 (1H, bs, NH), 9.26 (1H, bs, NH), 9.23 (1H, bs, NH), 8.25 (t, 1H, J=1.7Hz, phenyl-H-2), 7.91 (1 H, m, phenyl-H-4/6), 7.53 (1 H, m, phenyl-H-4/6), 7.43 (t, 1 H, J=7.9Hz, phenyl-H-5), 7.33 (1 H, d, J = 3.6Hz, furan-H-4), 7.30 (1 H, d, J =1.7Hz, pyrrole-H), 7.17 (1 H, d, J = 1.6Hz, pyrrole-H), 7.15 (1 H, d, J = 1.7Hz, pyrrole-H), 7.01 (1 H, d, J = 3.6Hz, furan-H-3), 6.81 (1 H, d, J = 1.7Hz, pyrrole-H), 3.96 (3H, s, NCH₃), 3.93 (3H, s, NCH₃), 3.88 (3H, s, OCH₃), 2.28 (4H, 2 x t, J = 7.0Hz, sidechain-H-2,4), 2.17 (6H, s, N[CH₃]₂), 1.78 (2H, p, J = 7.0Hz, sidechain-H-3); LCMS R_{T} = 2.26 min, (M⁺+1) = 575.

### (e) Ethyl 2-{4-[(4-{[4-(4-Dimethylamino-butyrylamino)-1-methyl-1H-pyrrole-2-carbonyl]-amino}-1-methyl-1H-pyrrole-2-carbonyl)-amino]-phenyl}-thiazole-4-carboxylate (57)

The yield was 0.035g (68%) of off white solid. ¹H NMR (d₆-Acetone) δ 9.48 (1H, bs, NH), 9.26 (1H, bs, NH), 9.24 (1 H, bs, NH), 8.36 (1 H, s, thiazole-H), 7.99 (4H, s, phenyl-H), 7.30 (d, 1H, J=1.7Hz, pyrrole-H), 7.17 (1H, d, J = 1.7Hz, pyrrole-H), 7.14 (1 H, d, J = 1.8Hz, pyrrole-H), 6.82 (1 H, d, J = 1.8Hz, pyrrole-H), 4.37 (2H, q, J = 7.1 Hz, OCH₂), 3.96 (3H, s, NCH₃), 3.93 (3H, s, NCH₃), 2.30 (4H, 2 x t, J = 7.0Hz, sidechain-H-2,4), 2.17 (6H, s, N[CH₃]₂), 1.77 (2H, p, J = 7.0Hz, sidechain-H-3), 1.38 (3H, t, J = 7.1 Hz, CH₂C*H*₃); LCMS R_{T} = 2.27 min, (M⁺+1) = 606.

### (f) Ethyl 2-{4-[(4-{[4-(4-Dimethylamino-butyrylamino)-1-methyl-1H-pyrrole-2-carbonyl]-amino}-1-methyl-1H-pyrrole-2-carbonyl)-amino]-phenyl}-4-methyl-thiazole-5-carboxylate (58)

The yield was 0.029g (57%) of off white solid. ¹H NMR (d₆-Acetone) δ 9.50 (1H, bs, NH), 9.25 (1H, bs, NH), 9.24 (1 H, bs, NH), 7.98 (4H, m, phenyl-H), 7.30 (1 H, d, J = 1.6Hz, pyrrole-H), 7.17 (1 H, d, J = 1.6Hz, pyrrole-H), 7.14 (1H, d, J = 1.7Hz, pyrrole-H), 6.82 (1H, d, J = 1.7Hz, pyrrole-H), 4.34 (2H, q, J = 7.1 Hz, OCH₂), 3.96 (3H, s, NCH₃), 3.93 (3H, s, NCH₃), 2.71 (3H, s, thiazole-CH₃), 2.30 (4H, 2xt, J = 7.0Hz, sidechain-H-2,4), 2.17 (6H, s, N[CH₃]₂), 1.78 (2H, p, J = 7.0Hz, sidechain-H-3), 1.36 (3H, t, J = 7.1 Hz, OCH₂CH₃); LCMS R_{T} = 2.47 min, (M⁺+1) = 620.

### (g) Methyl 4-{4-[(4-{[4-(4-Dimethylamino-butyrylamino)-1-methyl-1H-pyrrole-2-carbonyl]-amino}-1-methyl-1H-pyrrole-2-carbonyl)-amino]-phenyl}-1-methyl-1H-pyrrole-2-carboxylate (59)

The yield was 0.050g (97%) of off white solid. ¹H NMR (d₆-Acetone) δ 9.26 (1 H, bs, NH), 9.21 (1 H, bs, NH), 9.19 (1 H, bs, NH), 7.79 (2H, d, J = 8.7Hz, phenyl-H-2,6), 7.53 (2H, d, J = 8.7Hz, phenyl-H-3,5), 7.41 (1 H, d, J = 2.0Hz, phenylpyrrole-H), 7.27 (1 H, d, J = 1.7Hz, pyrrole-H), 7.19 (d, 1H, J=2.0Hz, phenylpyrrole-H), 7.17 (1 H, d, J = 1.7Hz, pyrrole-H), 7.05 (1 H, d, J = 1.8Hz, pyrrole-H), 6.81 (1 H, d, J = 1.8Hz, pyrrole-H), 3.96 (3H, s, NCH₃), 3.94 (3H, s, NCH₃), 3.92 (3H, s, NCH₃), 3.80 (3H, s, OCH₃), 2.30 (4H, 2xt, J = 7.1 Hz, sidechain-H-2,4), 2.17 (6H, s, N[CH₃]₂), 1.77 (3H, p, J = 7.0Hz, sidechain-H-3); LCMS R_{T} = 2.27 min, (M⁺+1) = 608.

### (h) Methyl 4-{4-[(4-{[4-(4-Dimethylamino-butyrylamino)-1-methyl-1H-pyrrole-2-carbonyl]-amino}-1-methyl-1H-pyrrole-2-carbonyl)-amino]-phenyl)-1-methyl-1H-imidazole-2-carboxylate (60)

The yield was 0.034g (66%) of off white solid. ¹H NMR (*d₆*-Acetone) δ 9.26 (1 H, bs, NH), 9.24 (1 H, bs, NH), 9.22 (1 H, bs, NH), 7.82 (4H, m, phenyl-H), 7.73 (1 H, s, imidazole-H), 7.28 (1 H, d, J = 1.6Hz, pyrrole-H), 7.17 (1 H, d, J = 1.6Hz, pyrrole-H), 7.07 (1 H, d, J = 1.7Hz, pyrrole-H), 6.81 (1 H, d, J = 1.6Hz, pyrrole-H), 4.05 (3H, s, NCH₃), 3.95 (3H, s, NCH₃), 3.93 (3H, s, NCH₃), 3.88 (3H, s, OCH₃), 2.29 (4H, 2xt, J = 7.0Hz, sidechain-H-2,4), 2.17 (6H, s, N[CH₃]₂), 1.78 (2H, p, J = 7.0Hz, sidechain-H-3); LCMS R_{T} = 1.92 min, (M⁺+1) = 587.

### (i) Ethyl 5-{4-[(4-{[4-(4-Dimethy/amino-butyry/amino)-1-methyl-1H-pyrro/e-2-carbony/]-amino}-1-methy/-1H-pyrro/e-2-carbonyl)-amino]-phenyl}-thiophene-2-carboxylato (61)

The reactionwas performed on 0.070g of Boc-protected amine. The yield was 0.060g (84%) of off white solid. ¹H NMR *(d₆-* Acetone) δ 9.41 (1H, bs, NH), 9.27 (1H, bs, NH), 9.24 (1H, bs, NH), 7.92 (2H, d, J = 8.7Hz, phenyl-H-2,6), 7.76 (1 H, d, J = 3.9Hz, thiophene-H-3), 7.72 (2H, d, J = 8.7Hz, phenyl-H-3,5), 7.47 (1 H, d, J = 3.9Hz, thiophene-H-4), 7.28 (1 H, d, J = 1.1 Hz, pyrrole-H), 7.17 (1 H, d, J = 1.2Hz, pyrrole-H), 7.11 (1 H, d, J = 1.2Hz, pyrrole-H), 6.82 (1 H, s, pyrrole-H), 4.33 (2H, q, J = 7.1 Hz, OCH₂), 3.95 (3H, s, NCH₃), 3.93 (3H, s, NCH₃), 2.30 (4H, 2xt, J = 7.0Hz, sidechain-H-2,4), 2.17 (6H, s, N[CH₃]₂), 1.77 (2H, p, J = 7.0Hz, sidechain-H-3), 1.36 (3H, t, J = 7.1 Hz, OCH₂CH₃); LCMS R_{T} = 2.48 min, (M⁺+1) = 605.

### Example 19 - PBD Coupled Biaryls

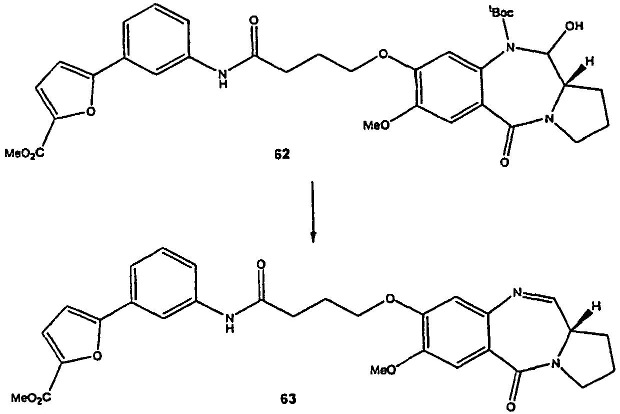

### (a) tert-Butyl 11-hydroxy-7-methoxy-8-{3-[3-(5-methoxycarbonyl-furan-2-yl)-phenylcarbamoyl]-propoxy}5-oxo-2,3,11,11a-tetrahydro-1H,5H-pyrrolo[2,1-c][1,4]benzodiazepine-10-carboxylate (62)

A solution of methyl 5-(3-nitrophenyl)-furan-5-carboxylate (0.220g, 0.9mmol) in ethyl acetate (50mL) was treated with a suspension of 10% palladium on charcoal (0.05g, 20% equiv.) The suspension was agitated under a hydrogen atmosphere (20psi) for 4 hours. The suspension was then filtered through celite and the solvent removed under vacuum. The resulting solid was dissolved in dry DMF (5mL) and the Boc protected PBD acid (0.402g, 0.9mmol, 1.0 equiv.) was added followed by EDCI (0.256g, 1.3mmol, 1.5 equiv.) and DMAP (0.131 g, 1.1 mmol, 1.2 equiv.). The reaction mixture was stirred for 48 hours then diluted with ethyl acetate (50mL) and washed with 10% citric acid (3 x 50mL) then saturated sodium hydrogen carbonate solution (3 x 50mL). The organic layer was dried over magnesium sulphate then concentrated under vacuum to give an off white foam, 0.387g (67%). An analytical sample (-0.2g) was purified by column chromatography (silica gel, eluted with EtOAc) to give 0.145g of **62** as a glassy solid. ¹H NMR (d₆-DMSO) δ 10.17 (1 H, bs, NH), 8.05 (1 H, m, phenyl-H-2), 7.67 (1H, m, phenyl-H-4/6), 7.50 (1 H, m, phenyl-H-4/6), 7.40 (2H, m, furan-H-3, H-6), 7.11 (1H,d, J = 3.6Hz, furan-H-4), 7.05 (1 H, s, phenyl-H-5), 6.70 (1 H, s, H-9), 6.38 (1 H, bs, OH), 5.41 (1 H, m, H-11), 4.07 (2H, m, sidechain-H-1), 3.84 (3H, s, OCH₃), 3.79 (3H, s, OCH₃), 3.46 (1 H, m, H-11 a), 3.36 (1 H, m, H-3), 3.24 (1 H, m, H-3), 2.67 (2H, m, sidechain-H-3), 2.55 (2H, m, sidechain-H-3), 2.33 (s, 1H), 2.10 (2H, m, H-1), 1.99 (2H, m, H-2), 1.27 (9H, s, O[CH₃]₃); MS (M⁺+1) = 650.

### (b) Methyl 5-{3-[4-(7-methoxy-5-oxo-1,2,3,11a-tetrahydro-5H-pyrrolo[2,1-c][1,4]benzodiazpein-8-yloxy)-butyrylamino]-phenyl}-furan-2-carboxylate (63)

A solution of the Boc-protected PBD (0.075g, 0.12mmol) in dichloromethane (1.5mL) was treated with trifluoroacetic acid and water (1.463mL:0.037mL). The reaction mixture was stirred for 1 hour at room temperature then poured into a mixture of ice/water (~40mL) and dichloromethane (10mL). The acid was neutralized by the careful addition of saturated sodium hydrogen carbonate (~25mL). The organic layer was separated and the aqueous layer washed with dichloromethane (3 x 15mL). The combined organic extracts were dried over magnesium sulphate then concentrated under vacuum to give 63 a pale yellow glassy solid, 0.059g (96%). ¹H NMR (*d₆*-DMSO) δ 10.16 (1 H, bs, NH), 8.05 (1 H, m, phenyl-H-2), 7.77 (1 H, d, J = 4.4Hz, H-11), 7.68 (1 H, m, phenyl-H-4/6), 7.50 (1 H, m, phenyl-H-4/6), 7.41 (2H, m, furan-H-3, H-6), 7.33 (1 H, m, phenyl-H-5), 7.11 (1 H, d, J = 3.6Hz, furan-H-4), 6.85 (1 H, s, H-9), 4.14 (2H, m, sidechain-H-1), 3.85 (3H, s, OCH₃), 3.81 (3H, s, OCH₃), 3.74 (1 H, m, H-11 a), 3.64 (1 H, m, H-3), 3.40 (1H, m, H-3), 2.53 (2H, m, sidechain-H-3), 2.27 (2H, m, H-1), 2.08 (2H, m, sidechain-H-2), 1.94 (2H, m, H-2).

### LC-MS Analysis

LC-MS analyses were performed using a Luna 3µ C8(2) column with a flow rate of 1.5mL/min and a linear gradient solvent system going from 95:5 solvent A:B at time 0 to 5:95 A:B at 4 minutes after sample injection then maintained at 5:95 until 7 minutes. Solvent A is 0.1 % formic acid in water, solvent B is 0.1 % formic acid in acetonitrile. The electrospray mass spectrometer was operated in switching mode to obtain both positive and negative ion spectra.

### Example 20 - DNA Footprinting

In order to assess the binding of test compounds to DNA, a footprinting study against the MS2 DNA sequence was carried out. The sequence is as follows:

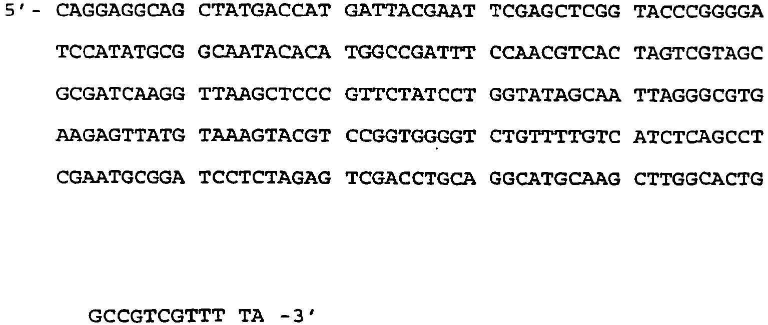

### and is derived from a bacteriophage.

The footprinting technique in the context of DNA allows the determination of binding sites for drugs or biological macromolecules. It relies on the fact that DNA is cleaved relatively non-specifically by free radicals (e.g. hydroxyl radical footprinting) or enzymes (e.g. DNase I footprinting). Thus if DNA is ³²P end labelled on one strand (so that it may be observed autoradiographically) and exposed to the cleavage agent for a certain time, then a laddering pattern may be seen when the resulting fragments are separated by gel electrophoresis (separation on the basis of size). If a compound which binds to DNA is added prior to the cleavage agent then this hinders access of the enzyme or radical to the DNA and blocks cleavage at the molecule binding site. Thus if the compound binds discretely then a specific cleavage block should be seen on the gel relative to the DNA not treated with compound, which is termed the 'footprint'. (see Figure 1)

Compound 9 produces an unusual profile in which there appears to be no specific footprinting activity, but conversely there is no clear coating event. There may be a structural aspect to the cleavage pattern seen.

## Claims

1. A compound of formula **I**:
Z'-CO-A-B-NH-Z (I)
wherein:
Z is an amino protecting group;
Z' is OH, a protected or activated hydroxyl group or Cl;
A is an optionally substituted C₅₋₆ arylene group;
B is an optionally substituted C₅₋₆ arylene group;
and wherein one of A and B is phenylene and the other of A and B is a C₅-heteroarylene group,
wherein when B is phenylene with an -NH- β to the bond between A and B, then -A-CO- is not:

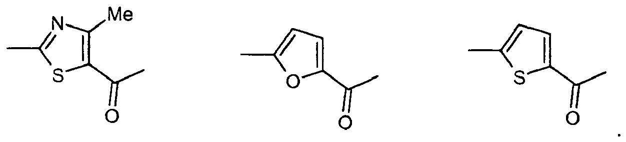

2. A compound according to claim 1, wherein Z' is a protected hydroxyl group.

3. A compound according to any one of claims 1 and 2, wherein A or B is a C₅ heteroaryl groups having one or two heteroatoms.

4. A compound of formula I:
Z'-CO-A-B-NH-Z (I)
wherein:
Z is H;
Z' is OH, a protected or activated hydroxyl group or Cl;
A is an optionally substituted C₅₋₆ arylene group;
B is an optionally substituted C₅₋₆ arylene group;
and wherein one of A and B is phenylene and the other of A and B is a C₅-heteroarylen group, and if B is phenylene, then A is not thiazolylene, furanylene or thiophenylene, and if B is pyridylene, then A is not phenylene.

5. A compound according to claim 4, wherein Z' is a protected hydroxyl group.

6. A compound according to any one of claims 4 and 5, wherein A or B is a C₅ heteroarylene group having one or two heteroatoms.

7. A compound of formula **IV**:
Z"-(T)ₙ-CO-(CH₂)_{q}-NR¹R² (IV)
comprsing a polyamido moiety, wherein:
Z" is OH or a protected hydroxy group;
each T is independently selected from units of formulae **II, III** or **V:**
-CO-A-B-NH- (II)
-CO-E-NH- (III)
-CO-(CH₂)_{q}-NH- (V)
and at least one T is a unit of formula II;
wherein:
A is an optionally substituted C₅₋₆ arylene group;
B is an optionally substituted C₅₋₆ arylene group;
E is either optionally substituted C₅₋₂₀ heteroarylene (G) or C₈₋₁₀ heteroarylene-C₅₋₂₀ arylene (K);
q' is from 1 to 3;
n is from 1 to 10;
q is from 1 to 3; and
R¹ and R² are independently selected from C₁₋₄ alkyl.

8. A compound according to claim 7, wherein A and B are independently selected from phenylene, and arylene groups derived from C₅ heteroaryl groups having one or two heteroatoms.

9. A compound comprising a polyamido moiety, itself comprising at least one unit of formula **II:**
-CO-A-B-NH- (II)
wherein:
A is an optionally substituted C₅₋₆ arylene group;
B is an optionally substituted C₅₋₆ arylene group;
and further comprising a pyrrolobenzodiazepine moiety of formula **VI**: 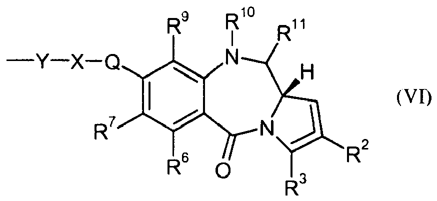 and salts, solvates, chemically protected forms, and prodrugs thereof, wherein:
the dotted lines indicate the optional presence of a double bond between C1 and C2 or C2 and C3;
R² and R³ are independently selected from -H, -OH, =O, =CH₂, -CN, -R, OR, halo, =CH-R, O-SO₂-R, CO₂R and COR;
R⁶, R⁷ and R⁹ are independently selected from H, R, OH, OR, SH, SR, NH₂, NHR, NRR', nitro, Me₃Sn and halo; where R and R' are independently selected from optionally substituted C₁₋₇ alkyl, C₃₋₂₀ heterocyclyl and C₅₋₂₀ aryl groups;
or R⁶ and R⁷ together form a group -O-(CH₂)ₚ-O-, where p is 1 or 2;
R¹⁰ is a nitrogen protecting group and R¹¹ is either O-R¹⁵, wherein R¹⁵ is a hydroxyl protecting group, or R¹⁵ is OH; or
R¹⁰ and R¹¹ together form a double bond between N10 and C11;
Q is selected from O, S, NH or a single bond;
X is a divalent group such that HY = R, or a single bond;
Y is either NH or C(=O).

10. A compound according to claim 9, wherein A and B are independently selected from phenylene, and arylene groups derived from C₅ heteroaryl groups having one or two heteroatoms.

11. A compound according to either claim 9 or claim 10, wherein R⁹ is H.

12. A compound according to any one of claims 9 to 11, wherein R⁶ is selected from H, OH, OR, SH, NH₂, nitro and halo.

13. A compound according to any one of claims 9 to 12, wherein R⁷ is independently selected from H, OR, SH, SR, NH₂, NHR, NRR' and halo.

14. A compound according to any one of claims 9 to 13, wherein R¹⁰ is BOC, Troc or alloc.

15. A compound according to any one of claims 9 to 14, wherein R¹⁵ is THP or a silyl oxygen protecting group.

16. A compound according to any one of claims 9 to 13, wherein R¹⁰ and R¹¹ together form a double bond between N10 and C11.

17. A compound according to any one of claims 9 to 16, wherein Q is NH, O or a single bond.

18. A compound according to any one of claims 9 to 17, wherein X is a single bond or C₁₋₇ alkylene.

19. A compound according to any one of claims 9 to 18, wherein R³ is H.

20. A compound according to any one of claims 9 to 19, wherein R² is R.

21. A compound according to any one of claims 7 to 20 for use in a method of therapy.

22. A pharmaceutical composition containing a compound of any one of claims 7 to 20, and a pharmaceutically acceptable carrier or diluent.

23. Use of a compound according to any one of claims 7 to (20) in the manufacture of a medicament for treating a proliferative disease.

## Patentansprüche

1. Verbindung der Formel I:
Z'-CO-A-B-NH-Z (I)
worin:
Z eine Amino-Schutzgruppe ist;
Z' OH, eine geschützte oder aktivierte Hydroxylgruppe oder Cl ist;
A eine gegebenenfalls substituierte C₅₋₆-Arylengruppe ist;
B eine gegebenenfalls substituierte C₅₋₆-Arylengruppe ist;
und worin eines von A und B Phenylen ist und das andere von A und B eine C₅-Heteroarylengruppe ist,
worin, wenn B Phenylen mit einem -NH- in β zur Bindung zwischen A und B ist, A-COnicht: 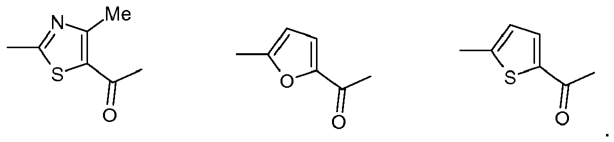 ist.

2. Verbindung nach Anspruch 1, worin Z' eine geschützte Hydroxylgruppe ist.

3. Verbindung nach Anspruch 1 oder 2, worin A oder B eine C₅-Heteroarylengruppe mit ein oder zwei Heteroatomen ist.

4. Verbindung der Formell:
Z'-CO-A-B-NH-Z (I)
worin:
Z H ist;
Z' OH, eine geschützte oder aktivierte Hydroxylgruppe oder Cl ist;
A eine gegebenenfalls substituierte C₅₋₆-Arylengruppe ist;
B eine gegebenenfalls substituierte C₅₋₆-Arylengruppe ist;
und worin eines von A und B Phenylen und das andere von A und B eine C₅ Heteroarylengruppe ist und, wenn B Phenylen ist, A nicht Thiazolylen, Furanylen oder Thiophenylen ist, und, wenn B Pyridylen ist, A nicht Phenylen ist.

5. Verbindung nach Anspruch 4, worin Z' eine geschützte Hydroxylgruppe ist.

6. Verbindung nach Anspruch 4 oder 5, worin A oder B eine C₅-Heteroarylengruppe mit ein oder zwei Heteroatomen ist.

7. Verbindung der Formel IV:
Z"-(T)ₙ-CO-(CH₂)_{q}-NR¹R² (IV)
die eine Polyamidogruppierung umfasst, worin:
Z" OH oder eine geschützte Hydroxygruppe ist;
die T jeweils unabhängig voneinander aus Einheiten der Formeln II, III und V ausgewählt sind:
-CO-A-B-NH- (II)
-CO-E-NH- (III)
-CO-(CH₂)_{q'}-NH- (V)
und zumindest einer T eine Einheit der Formel II ist;
worin:
A eine gegebenenfalls substituierte C₅₋₆-Arylengruppe ist;
B eine gegebenenfalls substituierte C₅-₆-Arylengruppe ist;
E entweder gegebenenfalls substitueirtes C₅-₂₀-Heteroarylen (G) oder C₈₋₁₀-Heteroarylen-C₅-₂₀-arylen (K) ist;
q' = 1 bis 3 ist;
n = 1 bis 10 ist;
q = 1 bis 3 ist; und
R¹ und R² unabhängig voneinander aus C₁₋₄-Alkyl ausgewählt sind.

8. Verbindung nach Anspruch 7, worin A und B unabhängig voneinander aus Phenylen- und Arylengruppen ausgewählt sind, die von C₅-Heteroarylgruppen mit ein oder zwei Heteroatomen abgeleitet sind.

9. Verbindung die eine Polyamidogruppierung umfasst, umfassend sich zumindest eine Einheit der Formel II:
-CO-A-B-NH- (II)
worin:
A eine gegebenenfalls substituierte C₅-₆-Arylengruppe ist;
B eine gegebenenfalls substituierte C₅₋₆-Arylengruppe ist;
und ferner umfassend eine Pyrrolobenzodiazepin-Gruppierung der Formel VI: 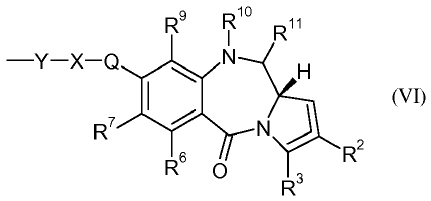 sowie Salze, Solvate, chemisch geschütze Formen und Prodrugs davon, worin:
die strichlierten Linien die optionale Gegenwart einer Doppelbindung zwischen C1 und C2 oder C2 und C3 anzeigen;
R² und R³ unabhängig voneinander aus -H, -OH, =O, =CH₂, -CN, -R, OR, Halogen, =CH-R, O-SO₂-R, CO₂R und COR ausgewählt sind;
R⁶, R⁷ und R⁹ unabhängig voneinander aus H, R, OH, OR, SH, SR, NH₂, NHR, NRR', Nitro, Me₃Sn und Halogen ausgewählt sind; worin R und R' unabhängig voneinander aus gegebenenfalls substituierten C₁₋₇-Alkyl-, C₃₋₂₀-Heterocyclyl- und C₅₋₂₀-Arylgruppen ausgewählt sind;
oder R⁶ und R⁷ gemeinsam eine Gruppe -O-(CH₂)ₚ-O- bilden, worin p = 1 oder 2 ist;
R¹⁰ eine Stickstoff-Schutzgruppe ist und R¹¹ entweder O-R¹⁵ ist, worin R¹⁵ eine Hydroxyl-Schutzgruppe ist, oder R¹⁵ OH ist; oder
R¹⁰ und R¹¹ gemeinsam eine Doppelbindung zwischen N10 und C11 bilden;
Q aus O, S, NH und einer Einfachbindung ausgewählt ist;
X eine solche zweiwertige Gruppe ist, dass NY = R ist, oder eine Einfachbindung ist;
Y entweder NH oder C(=O) ist.

10. Verbindung nach Anspruch 9, worin A und B unabhängig voneinander aus Phenylen- und Arylengruppen ausgewählt sind, die von C₅-Heteroarylgruppen mit ein oder zwei Heteroatomen abgeleitet sind.

11. Verbindung nach Anspruch 9 oder 10, worin R⁹ H ist.

12. Verbindung nach einem der Ansprüche 9 bis 11, worin R⁶ aus H, OH, OR, SH, NH₂, Nitro und Halogen ausgewählt ist.

13. Verbindung nach einem der Ansprüche 9 bis 12, worin R⁷ unabhängig aus H, OR, SH, SR, NH₂, NHR, NRR' und Halogen ausgewählt ist.

14. Verbindung nach einem der Ansprüche 9 bis 13, worin R¹⁰ BOC, Troc oder Alloc ist.

15. Verbindung nach einem der Ansprüche 9 bis 14, worin R¹⁵ THP oder eine Silyl-Sauerstoff-Schutzgruppe ist.

16. Verbindung nach einem der Ansprüche 9 bis 13, worin R¹⁰ und R¹¹ gemeinsam eine Doppelbindung zwischen N10 und C11 bilden.

17. Verbindung nach einem der Ansprüche 9 bis 16, worin Q NH, O oder eine Einfachbindung ist.

18. Verbindung nach einem der Ansprüche 9 bis 17, worin X eine Einfachbindung oder C₁₋₇Alkylen ist.

19. Verbindung nach einem der Ansprüche 9 bis 18, worin R³ H ist.

20. Verbindung nach einem der Ansprüche 9 bis 19, worin R² R ist.

21. Verbindung nach einem der Ansprüche 7 bis 20 zur Verwendung in einem Therapieverfahren.

22. Pharmazeutische Zusammensetzung, die eine Verbindung nach einem der Ansprüche 7 bis 20 und einen pharmazeutisch annehmbaren Träger oder Verdünner enthält.

23. Verwendung einer Verbindung nach einem der Ansprüche 7 bis 20 bei der Herstellung eines Medikaments zur Behandlung einer proliferativen Erkrankung.

## Revendications

1. Composé de formule I:
Z'-CO-A-B-NH-Z (I)
où:
Z est un groupe amino protecteur;
Z' est OH, un groupe hydroxyle protégé ou activé ou Cl;
A est un groupe arylène C₅₋₆ facultativement substitué;
B est un groupe arylène C₅₋₆ facultativement substitué;
et où l'un de A et B est phénylène et l'autre de A et B est un groupe hétéroarylène C₅; où quand B est phénylène avec un -NH- β à la liaison entre A et B, alors -A-CO- n'est pas:

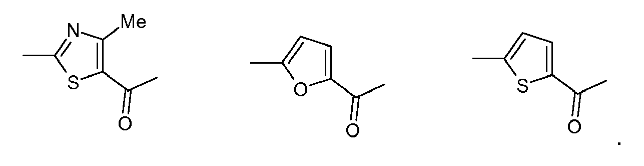

2. Composé selon la revendication 1, où Z' est un groupe hydroxyle protégé.

3. Composé selon l'une quelconque des revendications 1 et 2, où A ou B est un groupe hétéroarylène C₅ ayant un à deux hétéroatomes.

4. Composé de la formule I:
Z'-CO-A-B-NH-Z (I)
où:
Z est H;
Z' est OH, un groupe hydroxyle protégé ou activé ou Cl;
A est un groupe arylène C₅₋₆ facultativement substitué;
B est un groupe arylène C₅₋₆ facultativement substitué;
et où l'un de A et B est phénylène et l'autre de A et B est un groupe hétéroarylène C₅ et si B est phénylène, alors A n'est pas thiazolylène, furanylène ni thiophénylène et si B est pyridylène, alors A n'est pas phénylène.

5. Composé selon la revendication 4, où Z' est un groupe hydroxyle protégé.

6. Composé selon l'une quelconque des revendications 4 et 5, où A ou B est un groupe hétéroarylène C₅ ayant un ou deux hétéroatomes.

7. Composé de la formule II:
Z"-(T)ₙ-CO-(CH₂)_{q}-NR¹R² (IV)
comprenant une fraction polyamido, où:
Z" est OH ou un groupe hydroxyle protégé;
chaque T est indépendamment sélectionné parmi des unités de formule II, III ou V:
-CO-A-B-NH- (II)
-CO-E-NH- (III)
-CO-(CH₂)_{q'}-NH- (V)
et au moins une T est une unité de formule II;
où:
A est un groupe arylène C₅₋₆ facultativement substitué;
B est un groupe arylène C₅₋₆ facultativement substitué;
E est soit hétéroarylène C₅₋₂₀ facultativement substitué (G) ou hétéroarylène-C₈-₁₀ arylène-C₅₋₂₀ (K);
q' est de 1 à 3;
n est de 1 à 10;
q est de 1 à 3; et
R¹ et R² sont indépendamment sélectionnés parmi alkyle C₁₋₄.

8. Composé selon la revendication 7, où A et B sont indépendamment sélectionnés parmi des groupes phénylène et arylène dérivés de groupes hétéroaryles C₅ ayant un ou deux hétéroatomes.

9. Composé comprenant une fraction polyamido, lui-même comprenant au moins une unité de formule II:
-CO-A-B-NH- (II)
où:
A est un groupe arylène C₅₋₆ facultativement substitué;
B est un groupe arylène C₅₋₆ facultativement substitué;
et comprenant en outre une fraction de purrolobenzodiazépine de formule VI: 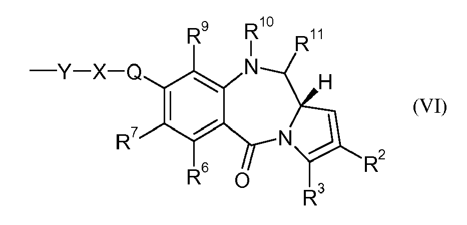
et les sels, solvates, formes chimiquement protégées, et promédicaments, où:
les lignes en pointillés indiquent la présence facultative d'une double liaison entre C1 et C2 ou C2 et C3;
R² et R³ sont indépendamment sélectionnés parmi -H, -OH, =O, =CH₂, -CN, -R, OR, halo, =CH-R, -O-SO₂-R, CO₂R et COR;
R⁶, R⁷ et R⁹ sont indépendamment sélectionnés parmi H, R, OH, SH, SR, NH₂, NHR, NRR', nitro, Me₃Sn et halo; où R et R' sont indépendamment sélectionnés parmi des groupes alkyle C₁₋₇, hétérocyclyle C₃₋₂₀ et aryle C₅₋₂₀ facultativement substitués;
ou bien R⁶ et R⁷ forment ensemble un groupe -O-(CH₂)ₚ-O-, où p est 1 ou 2;
R¹⁰ est un groupe protecteur d'azote et R¹¹ est soit O-R¹⁵, où
R¹⁵ est un groupe protecteur hydroxyle, ou bien R¹⁵ est OH; ou
R¹⁰ et R¹¹ forment ensemble une double liaison entre N10 et C11;
Q est sélectionné parmi O, S, NH ou une simple liaison;
X est un groupe divalent tel que HY = R, ou bien une simple liaison;
Y est soit NH ou C(=O).

10. Composé selon la revendication 9, où A et B sont indépendamment sélectionnés parmi des groupes phénylène et arylène dérivés de groupes hétéroaryles C₅ ayant un ou deux hétéroatomes.

11. Composé selon la revendication 9 ou la revendication 10, où R⁹ est H.

12. Composé selon l'une quelconque des revendications 9 à 11, où R⁶ est sélectionné parmi H, OH, OR, SH, NH₂, nitro et halo.

13. Composé selon l'une quelconque des revendications 9 à 12, où R⁷ est indépendamment sélectionné parmi H, OR, SH, SR, NH₂, NHR, NRR' et halo.

14. Composé selon l'une quelconque des revendications 9 à 13, où R¹⁰ est BOC, Troc ou alloc.

15. Composé selon l'une quelconque des revendications 9 à 14, où R¹⁵ est THP ou un groupe protecteur d'oxygène de silyle.

16. Composé selon l'une quelconque des revendications 9 à 13, où R¹⁰ et R¹¹ forment ensemble une double liaison entre N10 et C11.

17. Composé selon l'une quelconque des revendications 9 à 16, où Q est NH, O ou une simple liaison.

18. Composé selon l'une quelconque des revendications 9 à 17, où X est une simple liaison ou alkylène C₁₋₇.

19. Composé selon l'une quelconque des revendications 9 à 18, où R³ est H.

20. Composé selon l'une quelconque des revendications 9 à 19, où R² est R.

21. Composé selon l'une quelconque des revendications 7 à 20 à utiliser dans une méthode de thérapie.

22. Composition pharmaceutique contenant un composé selon l'une quelconque des revendications 7 à 20, et un support ou diluant pharmaceutiquement acceptable.

23. Utilisation d'un composé selon l'une quelconque des revendications 7 à 20 dans la fabrication d'un médicament pour le traitement d'une maladie proliférative.
